# EUROPEAN PATENT APPLICATION

(11) **EP 4 806 510 A2**
(43) Date of publication of application: **16.09.2026**
(21) Application number: 26167390.9
(22) Date of filing: 07.11.2023
(51) Int. Cl.: A61P 13/12

(54) **COMPOSITIONS AND METHODS OF TREATING CHILDHOOD ONSET IDIOPATHIC NEPHROTIC SYNDROME**

(30) Priority: 08.11.2022 US 202263423767 P
(62) Document number(s) of the earlier application(s) in accordance with Art. 76 EPC: 23821086.8 / 4 615 872
(71) Applicant: F. Hoffmann-La Roche AG, 4070 Basel (CH); Genentech, Inc., South San Francisco, CA 94080 (US)
(72) Inventor: LEHANE, Patricia Bernadette, Welwyn Garden City AL7 1TW (GB); OMACHI, Theodore Ari, South San Francisco, CA 94080 (US); CHENG, Ji, Mississauga L5N 5M8 (CA)
(74) Representative: Berendt, Frank Joachim Nathanael

(57) **Abstract**

The present disclosure provides methods for treating childhood-onset idiopathic nephrotic syndrome (INS), or reducing risk and/or frequency of relapse from childhood-onset INS, in an individual that is greater than or equal to 2 years of age and less than or equal to 25 years of age. In some embodiments, the methods comprise administering to the individual an effective amount of obinutuzumab.

## Description

### CROSS-REFERENCE TO RELATED APPLICATIONS

This application claims the priority benefit of U.S. Provisional Application Serial No. 63/423,767, filed November 8, 2022, which is incorporated herein by reference in its entirety.

### REFERENCE TO AN ELECTRONIC SEQUENCE LISTING

The contents of the electronic sequence listing (146392064240seqlist.xml; Size: 41,773 bytes; and Date of Creation: November 2, 2023) are incorporated herein by reference in their entirety.

### FIELD OF THE INVENTION

Provided herein are methods for treating childhood-onset idiopathic nephrotic syndrome (INS) in an individual (*e.g.,* an individual that is greater than or equal to 2 years of age and less than or equal to 25 years of age), or reducing risk and/or frequency of relapse in an individual with childhood-onset INS, by administering a type II anti-CD20 antibody.

### BACKGROUND

Childhood-onset idiopathic nephrotic syndrome (INS) is also known as primary nephrotic syndrome (excluding secondary causes) and encompasses minimal change disease (MCD) and focal and segmental glomerulosclerosis (FSGS). Although the disease is rare, the incidence of childhood-onset INS varies by ethnicity and region and is higher among certain ethnic groups, specifically South Asians and African-Americans, where the influence of genetic factors confers a higher risk, particularly for FSGS (Chanchlani and Parekh (2016) Front Pediatr 4:39). The disease is usually first diagnosed between 2 and 5 years of age (in 70% of patients with MCD), typically affects more boys than girls (2:1), and is defined by the presence of nephrotic-range proteinuria, edema, hyperlipidemia, and hypoalbuminemia (Noone et al. (2018) Lancet 392:61-74).

Patients with childhood-onset INS are initially treated with systemic oral corticosteroids. The frequency of relapses and response to corticosteroid treatment permits the classification of the disease into subtypes reflecting disease severity. These subtypes include "steroid-resistant nephrotic syndrome (underlying genetic cause)," "non/infrequent relapsing steroid sensitive nephrotic syndrome, " "FRNS," and "SDNS" (Noone et al. (2018) Lancet 392:61-74). In the latter two subtypes, patients receive multiple courses of steroids during episodes and often continue receiving steroid maintenance therapy to prevent further relapses. The optimal goal of treatment is maximal steroid sparing while limiting the relapse rate based on the patient's clinical response and drug-related adverse effects.

Childhood-onset INS recurs in more than 75% of patients and almost 50% of patients show frequent relapses or steroid dependency (Abdel-Hafez et al. (2017) J. Nephropathol 6:180-186). Among children whose disease cannot be controlled well with steroids, a number of steroid-sparing immunosuppressive agents (e.g., cyclophosphamide, levamisole, cyclosporin A, tacrolimus, MMF, and rituximab) have been shown to reduce the risk of relapse. However, high-quality head-to head data comparing these agents are limited (Mason et al. (2020) Clin. J. Am. Soc. Nephrol. 15:983-994).

The anti-CD20 monoclonal antibody rituximab was first described for the treatment of childhood-onset INS in 2004 (Benz et al. (2004) Pediatr Nephrol 19(7):794-797), and has since emerged as a promising unapproved treatment option in both children and adults with FRNS or SDNS. A number of investigator-initiated trials and case reports of its use have been described in the literature and clinical response rates vary (from 50%-85% of patients in renal remission at 12 months [no relapse]) and rituximab has been shown to reduce the annual rate of relapse in patients with FRNS and SDNS. However, patients who do not achieve complete remission at 12 months generally relapse between 8 and 9 months after rituximab treatment, and these relapses mostly occur in the context of B-cell recovery/reconstitution (Iijima et al. (2014) Lancet 384:1273-1281; Colucci et al. (2016) J. Am Soc Nephrol 27:1811-1822).

Delayed reconstitution of the memory B-cell pool is associated with a prolonged remission despite tapering or discontinuation of concomitant immunosuppression (Colucci et al. (2019) Front Immunol 10:1653). Obinutuzumab (GAZYVA^{®}, GAZYVARO^{®}) is a humanized, glycoengineered type II anti-CD20 antibody with enhanced depletion of B cells from peripheral blood and tissue relative to type I antibodies such as rituximab and ofatumumab. It is administered as an IV infusion. Consistent with its more potent B-cell depletion, obinutuzumab was superior to rituximab for the treatment of chronic lymphocytic leukemia (CLL) and follicular lymphoma (FL) in adults when administered in combination with standard chemotherapy and is currently approved worldwide in these indications. Obinutuzumab is also indicated for the treatment of patients with FL who did not respond to, or who progressed during or after treatment with, rituximab or a rituximab-containing regimen. In addition, obinutuzumab is currently in clinical development for adult and pediatric autoimmune diseases (lupus nephritis [LN], membranous nephropathy, and systemic lupus erythematosus [SLE]).

Despite current treatments for childhood-onset INS, patients remain at significant risk for impaired growth and other significant side effects of steroid-related toxicity. Given the frequent clinical relapse rates following the use of immunosuppressant therapies and the associated potential risk of end-stage kidney disease in childhood-onset INS, there remains an unmet need for approved steroid-sparing therapies with improved efficacy and better long-term outcomes.

All references cited herein, including patent applications and publications, are incorporated by reference in their entirety.

### SUMMARY

In certain aspects, provided herein is a method for treating childhood-onset INS in an individual, comprising administering to the individual at least a first antibody exposure to a type II anti-CD20 antibody and a second antibody exposure to the type II anti-CD20 antibody; wherein the second antibody exposure is not being provided until from about 18 weeks to about 26 weeks after the first antibody exposure; wherein the first antibody exposure comprises one or two doses of the type II anti-CD20 antibody, the first antibody exposure comprising: (a) a total exposure of between about 1800mg and about 2200mg of the type II anti-CD20 antibody, or (b) a total exposure of between about 36mg/kg and about 44mg/kg of the type II anti-CD20 antibody if the individual weighs less than 45kg; wherein the second antibody exposure comprises one or two doses of the type II anti-CD20 antibody, the second antibody exposure comprising: (c) a total exposure of between about 1800mg and about 2200mg of the type II anti-CD20 antibody, or (d) a total exposure of between about 36mg/kg and about 44mg/kg of the type II anti-CD20 antibody if the individual weighs less than 45kg; wherein the type II anti-CD20 antibody comprises a heavy chain comprising HVR-H1 sequence of SEQ ID NO:1, HVR-H2 sequence of SEQ ID NO:2, and HVR-H3 sequence of SEQ ID NO:3, and a light chain comprising HVR-L1 sequence of SEQ ID NO:4, HVR-L2 sequence of SEQ ID NO:5, and HVR-L3 sequence of SEQ ID NO:6; and wherein the individual is a human that is greater than or equal to 2 years of age and less than or equal to 25 years of age. Also provided herein is a method for preventing relapse, reducing risk of relapse, and/or reducing frequency of relapse in an individual with childhood-onset idiopathic nephrotic syndrome (INS), comprising administering to the individual at least a first antibody exposure to a type II anti-CD20 antibody and a second antibody exposure to the type II anti-CD20 antibody; wherein the second antibody exposure is not being provided until from about 18 weeks to about 26 weeks after the first antibody exposure; wherein the first antibody exposure comprises one or two doses of the type II anti-CD20 antibody, the first antibody exposure comprising: (a) a total exposure of between about 1800mg and about 2200mg of the type II anti-CD20 antibody, or (b) a total exposure of between about 36mg/kg and about 44mg/kg of the type II anti-CD20 antibody if the individual weighs less than 45kg; wherein the second antibody exposure comprises one or two doses of the type II anti-CD20 antibody, the second antibody exposure comprising: (c) a total exposure of between about 1800mg and about 2200mg of the type II anti-CD20 antibody, or (d) a total exposure of between about 36mg/kg and about 44mg/kg of the type II anti-CD20 antibody if the individual weighs less than 45kg; wherein the type II anti-CD20 antibody comprises a heavy chain comprising HVR-H1 sequence of SEQ ID NO:1, HVR-H2 sequence of SEQ ID NO:2, and HVR-H3 sequence of SEQ ID NO:3, and a light chain comprising HVR-L1 sequence of SEQ ID NO:4, HVR-L2 sequence of SEQ ID NO:5, and HVR-L3 sequence of SEQ ID NO:6; and wherein the individual is a human that is greater than or equal to 2 years of age and less than or equal to 25 years of age.

In some embodiments, the individual weighs greater than or equal to 45kg. In some embodiments, the first antibody exposure comprises a total exposure of between about 1800mg and about 2200mg of the type II anti-CD20 antibody; the second antibody exposure comprises a total exposure of between about 1800mg and about 2200mg of the type II anti-CD20 antibody; and the individual weighs greater than or equal to 45kg.

In some embodiments, the first antibody exposure comprises a first dose of between about 900mg and about 1100mg of the type II anti-CD20 antibody and a second dose of between about 900mg and about 1100mg of the type II anti-CD20 antibody. In some embodiments, the first antibody exposure comprises a first dose of between about 18mg/kg and about 22mg/kg of the type II anti-CD20 antibody and a second dose of between about 18mg/kg and about 22mg/kg of the type II anti-CD20 antibody, and wherein the individual weighs less than 45kg. In some embodiments, the first antibody exposure comprises a first dose of the type II anti-CD20 antibody and a second dose of the type II anti-CD20 antibody, and wherein the second dose of the first antibody exposure is not provided until from about 1.5 weeks to about 2.5 weeks after the first dose of the first antibody exposure. In some embodiments, the first antibody exposure comprises a first dose of the type II anti-CD20 antibody and a second dose of the type II anti-CD20 antibody, and wherein the second dose of the first antibody exposure is not provided until about 2 weeks after the first dose of the first antibody exposure. In some embodiments, the first dose of the first antibody exposure is about 1000mg of the type II anti-CD20 antibody. In some embodiments, the second dose of the first antibody exposure is about 1000mg of the type II anti-CD20 antibody. In some embodiments, the first dose of the first antibody exposure is about 20mg/kg of the type II anti-CD20 antibody, and wherein the individual weighs less than 45kg. In some embodiments, the second dose of the first antibody exposure is about 20mg/kg of the type II anti-CD20 antibody, and wherein the individual weighs less than 45kg. In some embodiments (*e.g.*, in which the dose(s) of the first antibody exposure are a flat dose), the individual weighs greater than or equal to 45kg.

In some embodiments, the second antibody exposure comprises a first dose of between about 18mg/kg and about 22mg/kg of the type II anti-CD20 antibody and a second dose of between about 18mg/kg and about 22mg/kg of the type II anti-CD20 antibody, and wherein the individual weighs less than 45kg. In some embodiments, the second antibody exposure comprises a first dose of the type II anti-CD20 antibody and a second dose of the type II anti-CD20 antibody, and wherein the second dose of the second antibody exposure is not provided until from about 1.5 weeks to about 2.5 weeks after the first dose of the second antibody exposure. In some embodiments, the second dose of the second antibody exposure is not provided until about 2 weeks after the first dose of the second antibody exposure. In some embodiments, the first dose of the second antibody exposure is about 1000mg of the type II anti-CD20 antibody. In some embodiments, the second dose of the second antibody exposure is about 1000mg of the type II anti-CD20 antibody. In some embodiments, the first dose of the second antibody exposure is about 20mg/kg of the type II anti-CD20 antibody, and wherein the individual weighs less than 45kg. In some embodiments, the second dose of the second antibody exposure is about 20mg/kg of the type II anti-CD20 antibody, and wherein the individual weighs less than 45kg. In some embodiments (*e.g.*, in which the dose(s) of the second antibody exposure are a flat dose), the individual weighs greater than or equal to 45kg.

In some embodiments, the first and/or the second antibody exposure is/are administered intravenously.

In some embodiments, the individual has or has been diagnosed with childhood-onset INS. In some embodiments, the individual has frequently relapsing nephrotic syndrome (FRNS). In some embodiments, the childhood-onset INS is steroid-dependent nephrotic syndrome (SDNS). In some embodiments, e.g., prior to administration of the first antibody exposure, the individual is in complete remission.

In some embodiments, the method further comprises administering to the individual an effective amount of a glucocorticoid or corticosteroid. In some embodiments, the glucocorticoid or corticosteroid comprises methylprednisolone. In some embodiments, the methylprednisolone is administered intravenously to the individual at a dose of 80mg. In some embodiments, e.g., if the individual weighs less than 45 kg, methylprednisolone is administered intravenously to the individual at a dose of 1.5mg/kg. In some embodiments, the glucocorticoid or corticosteroid comprises prednisone. In some embodiments, the method further comprises administering to the individual an effective amount of an antihistamine. In some embodiments, the antihistamine comprises diphenhydramine. In some embodiments, the diphenhydramine is administered orally at a dose of 0.5-1mg/kg, optionally to a maximum dose of 50mg. In some embodiments, the further comprises administering to the individual an effective amount of acetaminophen. In some embodiments, the acetaminophen is administered orally at a dose of 15mg/kg, optionally to a maximum dose of 1000mg.

In some embodiments, the method results in a sustained complete remission in the individual at 1 year. In some embodiments, the method results in a depletion of circulating peripheral B cells in the individual. In some embodiments, the circulating peripheral B cells are CD19+ B cells. In some embodiments, the B cells are naïve B cells (*e.g.,* CD19+ CD27- B cells), memory B cells (*e.g.,* CD19+ CD27+ B cells), or plasmablasts (*e.g.,* CD19+ CD27+ CD38++ B cells). In some embodiments, the B cells are CD19+CD3-CD14-CD33-CD56- cells. In some embodiments, after administration of the type II anti-CD20 antibody, B cells are depleted to a level such that circulating peripheral B cells are present in peripheral blood from the individual at about 5 cells/µL or fewer. In some embodiments, B cells are depleted to a level such that circulating peripheral B cells are present in peripheral blood from the individual at about 1 cells/µL or fewer. In some embodiments, B cells are depleted to a level such that circulating peripheral B cells are present in peripheral blood from the individual at about 0.5 cells/µL or fewer. In some embodiments, B cells are depleted to a level such that circulating peripheral B cells are present in peripheral blood from the individual the depletion is achieved after the first antibody exposure. In some embodiments, B cells are depleted to a level that is below the detectable limit using HSFC. In some embodiments, the HSFC has a lower limit of quantitation (LLOQ) for B cells of about 1.0 cells/µL or fewer, about 0.8 cells/µL or fewer, about 0.6 cells/µL or fewer, about 0.5 cells/µL or fewer, or 0.441 cells/µL or fewer. In some embodiments, B cell depletion is sustained for at least 52 weeks after the first dose of the first antibody exposure. In some embodiments, after administration of the type II anti-CD20 antibody, circulating peripheral B cells in the individual are depleted by at least about 90%, as compared to a corresponding measurement in the same individual before administration of the type II anti-CD20 antibody, or as compared to a corresponding measurement in an individual that has not received treatment with a type II anti-CD20 antibody.

In some embodiments, the first antibody exposure comprises two doses of 1000mg of the type II anti-CD20 antibody on days 1 and 15 of treatment; the second antibody exposure comprises two doses of 1000mg of the type II anti-CD20 antibody on days 168 and 182 of treatment; and the type II anti-CD20 antibody is obinutuzumab. In some embodiments, the first antibody exposure comprises two doses of 20mg/kg of the type II anti-CD20 antibody on days 1 and 15 of treatment; the second antibody exposure comprises two doses of 20mg/kg of the type II anti-CD20 antibody on days 168 and 182 of treatment; the type II anti-CD20 antibody is obinutuzumab; and the individual weighs less than 45kg. In some embodiments, the first antibody exposure comprises two doses of 1000mg of the type II anti-CD20 antibody on weeks 0 and 2 of treatment; the second antibody exposure comprises two doses of 1000mg of the type II anti-CD20 antibody on weeks 24 and 26 of treatment; and the type II anti-CD20 antibody is obinutuzumab. In some embodiments, the first antibody exposure comprises two doses of 20mg/kg of the type II anti-CD20 antibody on weeks 0 and 2 of treatment; the second antibody exposure comprises two doses of 20mg/kg of the type II anti-CD20 antibody on weeks 24 and 26 of treatment; the type II anti-CD20 antibody is obinutuzumab; and the individual weighs less than 45kg. In some embodiments (*e.g.*, in which the doses of the antibody exposures are flat doses), the individual weighs greater than or equal to 45kg.

In certain aspects, provided herein is a method for treating, or reducing risk and/or frequency of relapse of, childhood-onset INS in an individual, comprising administering intravenously to the individual a first and a second antibody exposure to a type II anti-CD20 antibody; wherein the first antibody exposure comprises two doses of 1000mg of the type II anti-CD20 antibody on weeks 0 and 2 of treatment; wherein the second antibody exposure comprises two doses of 1000mg of the type II anti-CD20 antibody on weeks 24 and 26 of treatment; wherein the type II anti-CD20 antibody comprises a heavy chain comprising HVR-H1 sequence of SEQ ID NO:1, HVR-H2 sequence of SEQ ID NO:2, and HVR-H3 sequence of SEQ ID NO:3, and a light chain comprising HVR-L1 sequence of SEQ ID NO:4, HVR-L2 sequence of SEQ ID NO:5, and HVR-L3 sequence of SEQ ID NO:6; wherein the individual is a human that is greater than or equal to 2 years of age and less than or equal to 25 years of age; and wherein the individual weighs greater than or equal to 45kg. In certain aspects, provided herein is a method for treating, or reducing risk and/or frequency of relapse of, childhood-onset INS in an individual, comprising administering intravenously to the individual a first and a second antibody exposure to a type II anti-CD20 antibody; wherein the first antibody exposure comprises two doses of 20mg/kg of the type II anti-CD20 antibody on weeks 0 and 2 of treatment; wherein the second antibody exposure comprises two doses of 20mg/kg of the type II anti-CD20 antibody on weeks 24 and 26 of treatment; wherein the type II anti-CD20 antibody comprises a heavy chain comprising HVR-H1 sequence of SEQ ID NO:1, HVR-H2 sequence of SEQ ID NO:2, and HVR-H3 sequence of SEQ ID NO:3, and a light chain comprising HVR-L1 sequence of SEQ ID NO:4, HVR-L2 sequence of SEQ ID NO:5, and HVR-L3 sequence of SEQ ID NO:6; wherein the individual is a human that is greater than or equal to 2 years of age and less than or equal to 25 years of age; and wherein the individual weighs less than 45kg. In certain aspects, provided herein is a method for treating, or reducing risk and/or frequency of relapse of, childhood-onset INS in an individual, comprising administering intravenously to the individual a first and a second antibody exposure to a type II anti-CD20 antibody; wherein the first antibody exposure comprises two doses of 1000mg of the type II anti-CD20 antibody on days 1 and 15 of treatment; wherein the second antibody exposure comprises two doses of 1000mg of the type II anti-CD20 antibody on days 168 and 182 of treatment; wherein the type II anti-CD20 antibody comprises a heavy chain comprising HVR-H1 sequence of SEQ ID NO:1, HVR-H2 sequence of SEQ ID NO:2, and HVR-H3 sequence of SEQ ID NO:3, and a light chain comprising HVR-L1 sequence of SEQ ID NO:4, HVR-L2 sequence of SEQ ID NO:5, and HVR-L3 sequence of SEQ ID NO:6; wherein the individual is a human that is greater than or equal to 2 years of age and less than or equal to 25 years of age; and wherein the individual weighs greater than or equal to 45kg. In certain aspects, provided herein is a method for treating, or reducing risk and/or frequency of relapse of, childhood-onset INS in an individual, comprising administering intravenously to the individual a first and a second antibody exposure to a type II anti-CD20 antibody; wherein the first antibody exposure comprises two doses of 20mg/kg of the type II anti-CD20 antibody on days 1 and 15 of treatment; wherein the second antibody exposure comprises two doses of 20mg/kg of the type II anti-CD20 antibody on days 168 and 182 of treatment; wherein the type II anti-CD20 antibody comprises a heavy chain comprising HVR-H1 sequence of SEQ ID NO:1, HVR-H2 sequence of SEQ ID NO:2, and HVR-H3 sequence of SEQ ID NO:3, and a light chain comprising HVR-L1 sequence of SEQ ID NO:4, HVR-L2 sequence of SEQ ID NO:5, and HVR-L3 sequence of SEQ ID NO:6; wherein the individual is a human that is greater than or equal to 2 years of age and less than or equal to 25 years of age; and wherein the individual weighs less than 45kg. In some embodiments, the type II anti-CD20 antibody is obinutuzumab.

In some embodiments of the methods described herein, the type II anti-CD20 antibody is a humanized antibody. In some embodiments, the type II anti-CD20 antibody is afucosylated. In some embodiments, the heavy chain of the type II anti-CD20 antibody comprises a heavy chain variable region comprising the amino acid sequence of SEQ ID NO:7. In some embodiments, the light chain of the type II anti-CD20 antibody comprises a light chain variable region comprising the amino acid sequence of SEQ ID NO:8. In some embodiments, the type II anti-CD20 antibody comprises the heavy chain variable region comprising the amino acid sequence of SEQ ID NO:7, and the light chain variable region comprising the amino acid sequence of SEQ ID NO:8. In some embodiments, the type II anti-CD20 antibody comprises a heavy chain comprising the amino acid sequence of SEQ ID NO: 9 and a light chain comprising the amino acid sequence of SEQ ID NO: 10. In some embodiments, the type II anti-CD20 antibody is obinutuzumab.

In some embodiments, the methods further comprise administering prednisone to the individual (*e.g.*, orally). In some embodiments, oral prednisone is administered to the individual at a dose of 0.5-1mg/kg/day with a maximum of 60mg/day. In some embodiments, oral prednisone is administered to the individual at a dose of 0.5-1mg/kg/day until week 2, then tapered to a dose of 5mg/day by week 24 of treatment. In some embodiments, oral prednisone is administered to the individual at a dose of 0.5-2mg/kg/day with a maximum of 60mg/day. In some embodiments, oral prednisone is administered to the individual at a dose of 0.5-2mg/kg/day until week 2, then tapered to a dose of 5mg/day by week 24 of treatment. In some embodiments, the methods further comprise administering to the individual methylprednisolone by intravenous (IV) infusion at weeks 0, 2, 24, 26, and 52 of treatment, *e.g.*, prior to administration of the type II anti-CD20 antibody. In some embodiments, 80mg methylprednisolone is administered to the individual if the individual weighs greater than or equal to 45kg. In some embodiments, 1.5mg/kg methylprednisolone is administered to the individual if the individual weighs less than 45kg.

In certain aspects, provided herein is a kit for treating, or reducing risk and/or frequency of relapse of, childhood-onset INS in an individual, comprising: a container comprising a type II anti-CD20 antibody, wherein the type II anti-CD20 antibody comprises a heavy chain comprising HVR-H1 sequence of SEQ ID NO:1, HVR-H2 sequence of SEQ ID NO:2, and HVR-H3 sequence of SEQ ID NO:3, and a light chain comprising HVR-L1 sequence of SEQ ID NO:4, HVR-L2 sequence of SEQ ID NO:5, and HVR-L3 sequence of SEQ ID NO:6; a package insert with instructions for using the type II anti-CD20 antibody in any of the methods described above and herein.

In certain aspects, provided herein is a type II anti-CD20 antibody (*e.g.*, obinutuzumab) for use in any of the methods described above and herein.

It is to be understood that one, some, or all of the properties of the various embodiments described herein may be combined to form other embodiments of the present invention. These and other aspects of the invention will become apparent to one of skill in the art. These and other embodiments of the invention are further described by the detailed description that follows.

### BRIEF DESCRIPTION OF THE FIGURE

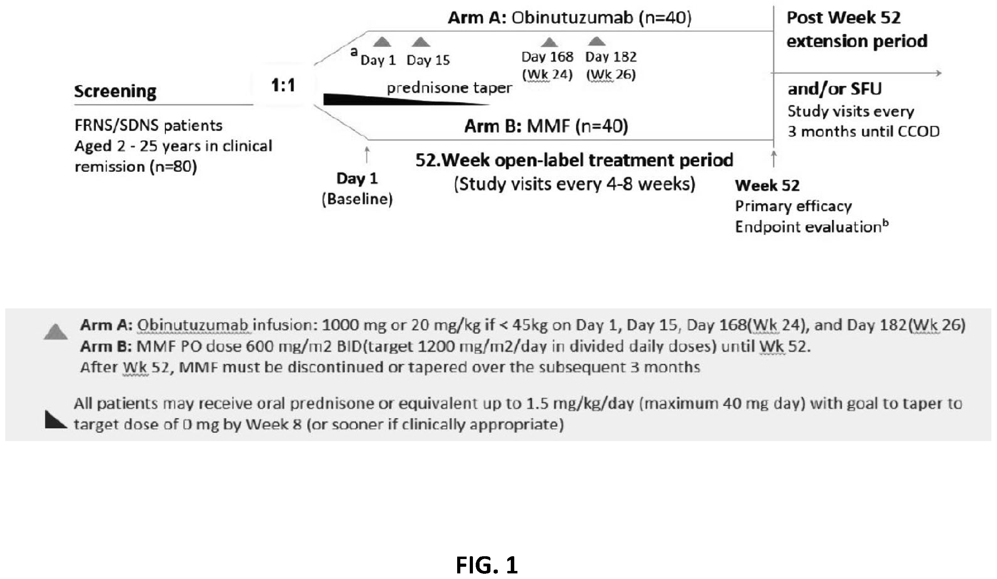

**FIG. 1** provides a schematic diagram of a controlled study of the use of the type II anti-CD20 antibody obinutuzumab in treating childhood-onset INS (*e.g.*, frequently relapsing nephrotic syndrome or steroid-dependent nephrotic syndrome) in patients aged ≥ 2-25 years. BID = twice a day; CCOD = common close out date for the primary analysis; FRNS = Frequent Relapsing Nephrotic Syndrome; MMF = Mycophenolate Mofetil; PO = by mouth; orally; SDNS = Steroid Dependent Nephrotic Syndrome; SFU = safety follow up; Wk = week. ^{a} Administration of the first dose of study treatment (Day 1) should occur within 24 hours following the baseline assessments. However, administration up to 72 hours is allowed when necessary. The second infusion should occur on Day 15 ± 1 day. ^{b} The primary efficacy endpoint of Proportion of Participants with Sustained Complete Remission at one year is measured at Week 52.

### DETAILED DESCRIPTION

Childhood-onset idiopathic nephrotic syndrome (INS) is also known as primary nephrotic syndrome (excluding secondary causes) and encompasses minimal change disease (MCD) and focal and segmental glomerulosclerosis (FSGS). The disease is usually first diagnosed between 2 and 5 years of age (in 70% of patients with MCD), typically affects more boys than girls (2:1), and is defined by the presence of nephrotic-range proteinuria, edema, hyperlipidemia, and hypoalbuminemia (Noone et al. (2018) Lancet 392:61-74). Patients with childhood-onset INS are initially treated with systemic oral corticosteroids. However, Childhood-onset INS recurs in more than 75% of patients and almost 50% of patients show frequent relapses or steroid dependency (Abdel-Hafez et al. (2017) J. Nephropathol 6:180-186). Despite current treatments, patients remain at significant risk for impaired growth and other significant side effects of steroid-related toxicity. As such, there continues to be a need for safer and more effective treatments for childhood-onset INS (*e.g.*, FRNS and/or SDNS).

In one aspect, provided herein are methods for treating childhood-onset INS in an individual, comprising administering to the individual at least a first antibody exposure to a type II anti-CD20 antibody and a second antibody exposure to the type II anti-CD20 antibody; wherein the second antibody exposure is not being provided until from about 18 weeks to about 26 weeks after the first antibody exposure; wherein the first antibody exposure comprises one or two doses of the type II anti-CD20 antibody, the first antibody exposure comprising: (a) a total exposure of between about 1800mg and about 2200mg of the type II anti-CD20 antibody, or (b) a total exposure of between about 36mg/kg and about 44mg/kg of the type II anti-CD20 antibody if the individual weighs less than 45kg; wherein the second antibody exposure comprises one or two doses of the type II anti-CD20 antibody, the second antibody exposure comprising: (c) a total exposure of between about 1800mg and about 2200mg of the type II anti-CD20 antibody, or (d) a total exposure of between about 36mg/kg and about 44mg/kg of the type II anti-CD20 antibody if the individual weighs less than 45kg; wherein the type II anti-CD20 antibody comprises a heavy chain comprising HVR-H1 sequence of SEQ ID NO:1, HVR-H2 sequence of SEQ ID NO:2, and HVR-H3 sequence of SEQ ID NO:3, and a light chain comprising HVR-L1 sequence of SEQ ID NO:4, HVR-L2 sequence of SEQ ID NO:5, and HVR-L3 sequence of SEQ ID NO:6; and wherein the individual is a human that is greater than or equal to 2 years of age and less than or equal to 25 years of age. In another aspect, provided herein are methods for reducing risk and/or frequency of relapse in an individual with childhood-onset idiopathic nephrotic syndrome (INS), comprising administering to the individual at least a first antibody exposure to a type II anti-CD20 antibody and a second antibody exposure to the type II anti-CD20 antibody; wherein the second antibody exposure is not being provided until from about 18 weeks to about 26 weeks after the first antibody exposure; wherein the first antibody exposure comprises one or two doses of the type II anti-CD20 antibody, the first antibody exposure comprising: (a) a total exposure of between about 1800mg and about 2200mg of the type II anti-CD20 antibody, or (b) a total exposure of between about 36mg/kg and about 44mg/kg of the type II anti-CD20 antibody if the individual weighs less than 45kg; wherein the second antibody exposure comprises one or two doses of the type II anti-CD20 antibody, the second antibody exposure comprising: (c) a total exposure of between about 1800mg and about 2200mg of the type II anti-CD20 antibody, or (d) a total exposure of between about 36mg/kg and about 44mg/kg of the type II anti-CD20 antibody if the individual weighs less than 45kg; wherein the type II anti-CD20 antibody comprises a heavy chain comprising HVR-H1 sequence of SEQ ID NO:1, HVR-H2 sequence of SEQ ID NO:2, and HVR-H3 sequence of SEQ ID NO:3, and a light chain comprising HVR-L1 sequence of SEQ ID NO:4, HVR-L2 sequence of SEQ ID NO:5, and HVR-L3 sequence of SEQ ID NO:6; and wherein the individual is a human that is greater than or equal to 2 years of age and less than or equal to 25 years of age.

### I. General Techniques

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3d edition (2001) Cold Spring Harbor Laboratory Press, Cold Spring Harbor, N.Y.; Current Protocols in Molecular Biology (F.M. Ausubel, et al. eds., (2003)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, A Laboratory Manual, and Animal Cell Culture (R.I. Freshney, ed. (1987)); Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney), ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Handbook of Experimental Immunology (D.M. Weir and C.C. Blackwell, eds.); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 1993).

### II. Definitions

The term "Childhood-onset idiopathic nephrotic syndrome (INS)" refers to an idiopathic nephrotic syndrome typically first diagnosed between 2 and 5 years of age that encompasses minimal change disease (MCD) and focal and segmental glomerulosclerosis (FSGS) and is also known as primary nephrotic syndrome (excluding secondary causes).

The term "antibody" includes monoclonal antibodies (including full length antibodies which have an immunoglobulin Fc region), antibody compositions with polyepitopic specificity, multispecific antibodies (*e.g*., bispecific antibodies, diabodies, and single-chain molecules, as well as antibody fragments (*e.g*., Fab, F(ab')₂, and Fv). The term "immunoglobulin" (Ig) is used interchangeably with "antibody" herein.

The basic 4-chain antibody unit is a heterotetrameric glycoprotein composed of two identical light (L) chains and two identical heavy (H) chains. An IgM antibody consists of 5 of the basic heterotetramer units along with an additional polypeptide called a J chain, and contains 10 antigen binding sites, while IgA antibodies comprise from 2-5 of the basic 4-chain units which can polymerize to form polyvalent assemblages in combination with the J chain. In the case of IgGs, the 4-chain unit is generally about 150,000 daltons. Each L chain is linked to an H chain by one covalent disulfide bond, while the two H chains are linked to each other by one or more disulfide bonds depending on the H chain isotype. Each H and L chain also has regularly spaced intrachain disulfide bridges. Each H chain has at the N-terminus, a variable domain (V_{H}) followed by three constant domains (C_{H}) for each of the α and γ chains and four C_{H} domains for µ and ε isotypes. Each L chain has at the N-terminus, a variable domain (V_{L}) followed by a constant domain at its other end. The V_{L} is aligned with the V_{H} and the C_{L} is aligned with the first constant domain of the heavy chain (C_{H}1). Particular amino acid residues are believed to form an interface between the light chain and heavy chain variable domains. The pairing of a V_{H} and V_{L} together forms a single antigen-binding site. For the structure and properties of the different classes of antibodies, see *e.g.,* Basic and Clinical Immunology, 8th Edition, Daniel P. Sties, Abba I. Terr and Tristram G. Parsolw (eds), Appleton & Lange, Norwalk, CT, 1994, page 71 and Chapter 6. The L chain from any vertebrate species can be assigned to one of two clearly distinct types, called kappa and lambda, based on the amino acid sequences of their constant domains. Depending on the amino acid sequence of the constant domain of their heavy chains (CH), immunoglobulins can be assigned to different classes or isotypes. There are five classes of immunoglobulins: IgA, IgD, IgE, IgG and IgM, having heavy chains designated α, δ, ε, γ and µ, respectively. The γ and α classes are further divided into subclasses on the basis of relatively minor differences in the CH sequence and function, *e.g.*, humans express the following subclasses: IgG1, IgG2A, IgG2B, IgG3, IgG4, IgA1 and IgA2.

The "variable region" or "variable domain" of an antibody refers to the amino-terminal domains of the heavy or light chain of the antibody. The variable domains of the heavy chain and light chain may be referred to as "*VH*" and "*VL*", respectively. These domains are generally the most variable parts of the antibody (relative to other antibodies of the same class) and contain the antigen binding sites.

The term "variable" refers to the fact that certain segments of the variable domains differ extensively in sequence among antibodies. The V domain mediates antigen binding and defines the specificity of a particular antibody for its particular antigen. However, the variability is not evenly distributed across the entire span of the variable domains. Instead, it is concentrated in three segments called hypervariable regions (HVRs) both in the light-chain and the heavy chain variable domains. The more highly conserved portions of variable domains are called the framework regions (FR). The variable domains of native heavy and light chains each comprise four FR regions, largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen binding site of antibodies (see Kabat et al., Sequences of Immunological Interest, Fifth Edition, National Institute of Health, Bethesda, MD (1991)). The constant domains are not involved directly in the binding of antibody to an antigen, but exhibit various effector functions, such as participation of the antibody in antibody-dependent cellular toxicity.

The term "monoclonal antibody" as used herein refers to an antibody obtained from a population of substantially homogeneous antibodies, *i.e*., the individual antibodies comprising the population are identical except for possible naturally occurring mutations and/or post-translation modifications (*e.g.*, isomerizations, amidations) that may be present in minor amounts. Monoclonal antibodies are highly specific, being directed against a single antigenic site. In contrast to polyclonal antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (*e.g.*, Kohler and Milstein., Nature, 256:495-97 (1975); Hongo et al., Hybridoma, 14 (3): 253-260 (1995), Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981)), recombinant DNA methods (see, *e.g.*, U.S. Patent No. 4,816,567), phage-display technologies (see, *e.g.*, Clackson et al., Nature, 352: 624-628 (1991); Marks et al., J. Mol. Biol. 222: 581-597 (1992); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132 (2004), and technologies for producing human or human-like antibodies in animals that have parts or all of the human immunoglobulin loci or genes encoding human immunoglobulin sequences (see, *e.g.*, WO 1998/24893; WO 1996/34096; WO 1996/33735; WO 1991/10741; Jakobovits et al., Proc. Natl. Acad. Sci. USA 90: 2551 (1993); Jakobovits et al., Nature 362: 255-258 (1993); Bruggemann et al., Year in Immunol. 7:33 (1993); U.S. Patent Nos. 5,545,807; 5,545,806; 5,569,825; 5,625,126; 5,633,425; and 5,661,016; Marks et al., Bio/Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996); and Lonberg and Huszar, Intern. Rev. Immunol. 13: 65-93 (1995).

The term "naked antibody" refers to an antibody that is not conjugated to a cytotoxic moiety or radiolabel.

The terms "full-length antibody," "intact antibody" or "whole antibody" are used interchangeably to refer to an antibody in its substantially intact form, as opposed to an antibody fragment. Specifically whole antibodies include those with heavy and light chains including an Fc region. The constant domains may be native sequence constant domains (*e.g.*, human native sequence constant domains) or amino acid sequence variants thereof. In some cases, the intact antibody may have one or more effector functions.

An "antibody fragment" comprises a portion of an intact antibody, preferably the antigen binding and/or the variable region of the intact antibody. Examples of antibody fragments include Fab, Fab', F(ab')₂ and Fv fragments; diabodies; linear antibodies (see U.S. Patent 5,641,870, Example 2; Zapata et al., Protein Eng. 8(10): 1057-1062 [1995]); single-chain antibody molecules and multispecific antibodies formed from antibody fragments. Papain digestion of antibodies produced two identical antigen-binding fragments, called "Fab" fragments, and a residual "Fc" fragment, a designation reflecting the ability to crystallize readily. The Fab fragment consists of an entire L chain along with the variable region domain of the H chain (V_{H}), and the first constant domain of one heavy chain (C_{H}1). Each Fab fragment is monovalent with respect to antigen binding*, i.e*., it has a single antigen-binding site. Pepsin treatment of an antibody yields a single large F(ab')₂ fragment which roughly corresponds to two disulfide linked Fab fragments having different antigen-binding activity and is still capable of cross-linking antigen. Fab' fragments differ from Fab fragments by having a few additional residues at the carboxy terminus of the C_{H}1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The Fc fragment comprises the carboxy-terminal portions of both H chains held together by disulfides. The effector functions of antibodies are determined by sequences in the Fc region, the region which is also recognized by Fc receptors (FcR) found on certain types of cells.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) has the ability to recognize and bind antigen, although at a lower affinity than the entire binding site.

"Single-chain Fv" also abbreviated as "sFv" or "scFv" are antibody fragments that comprise the V_{H} and V_{L} antibody domains connected into a single polypeptide chain. Preferably, the sFv polypeptide further comprises a polypeptide linker between the V_{H} and V_{L} domains which enables the sFv to form the desired structure for antigen binding. For a review of the sFv, see Pluckthun in The Pharmacology of Monoclonal Antibodies, vol. 113, Rosenburg and Moore eds., Springer-Verlag, New York, pp. 269-315 (1994).

"Functional fragments" of the antibodies of the invention comprise a portion of an intact antibody, generally including the antigen binding or variable region of the intact antibody or the Fc region of an antibody which retains or has modified FcR binding capability. Examples of antibody fragments include linear antibody, single-chain antibody molecules and multispecific antibodies formed from antibody fragments.

The term "diabodies" refers to small antibody fragments prepared by constructing sFv fragments (see preceding paragraph) with short linkers (about 5-10) residues) between the V_{H} and V_{L} domains such that inter-chain but not intra-chain pairing of the V domains is achieved, thereby resulting in a bivalent fragment, *i.e*., a fragment having two antigen-binding sites. Bispecific diabodies are heterodimers of two "crossover" sFv fragments in which the V_{H} and V_{L} domains of the two antibodies are present on different polypeptide chains. Diabodies are described in greater detail in, for example, EP 404,097; WO 93/11161; Hollinger et al., Proc. Natl. Acad. Sci. USA 90: 6444-6448 (1993).

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is(are) identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)). Chimeric antibodies of interest herein include PRIMATIZED^{®} antibodies wherein the antigen-binding region of the antibody is derived from an antibody produced by, *e.g.*, immunizing macaque monkeys with an antigen of interest. As used herein, "humanized antibody" is used a subset of "chimeric antibodies."

"Humanized" forms of non-human (*e.g*., murine) antibodies are chimeric antibodies that contain minimal sequence derived from non-human immunoglobulin. In one embodiment, a humanized antibody is a human immunoglobulin (recipient antibody) in which residues from an HVR (hereinafter defined) of the recipient are replaced by residues from an HVR of a non-human species (donor antibody) such as mouse, rat, rabbit or non-human primate having the desired specificity, affinity, and/or capacity. In some instances, framework ("FR") residues of the human immunoglobulin are replaced by corresponding non-human residues. Furthermore, humanized antibodies may comprise residues that are not found in the recipient antibody or in the donor antibody. These modifications may be made to further refine antibody performance, such as binding affinity. In general, a humanized antibody will comprise substantially all of at least one, and typically two, variable domains, in which all or substantially all of the hypervariable loops correspond to those of a non-human immunoglobulin sequence, and all or substantially all of the FR regions are those of a human immunoglobulin sequence, although the FR regions may include one or more individual FR residue substitutions that improve antibody performance, such as binding affinity, isomerization, immunogenicity, *etc*. The number of these amino acid substitutions in the FR are typically no more than 6 in the H chain, and in the L chain, no more than 3. The humanized antibody optionally will also comprise at least a portion of an immunoglobulin constant region (Fc), typically that of a human immunoglobulin. For further details, see, *e.g.*, Jones et al., Nature 321:522-525 (1986); Riechmann et al., Nature 332:323-329 (1988); and Presta, Curr. Op. Struct. Biol. 2:593-596 (1992). See also, for example, Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994); and U.S. Pat. Nos. 6,982,321 and 7,087,409.

A "human antibody" is an antibody that possesses an amino-acid sequence corresponding to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, *e.g*., immunized xenomice (see, *e.g.*, U.S. Pat. Nos. 6,075,181 and 6,150,584 regarding XENOMOUSE^{™} technology). See also, for example, Li et al., Proc. Natl. Acad. Sci. USA, 103:3557-3562 (2006) regarding human antibodies generated via a human B-cell hybridoma technology.

The term "hypervariable region," "HVR," or "HV," when used herein refers to the regions of an antibody variable domain which are hypervariable in sequence and/or form structurally defined loops. Generally, antibodies comprise six HVRs; three in the VH (H1, H2, H3), and three in the VL (L1, L2, L3). In native antibodies, H3 and L3 display the most diversity of the six HVRs, and H3 in particular is believed to play a unique role in conferring fine specificity to antibodies. See, e.g., Xu et al., Immunity 13:37-45 (2000); Johnson and Wu, in Methods in Molecular Biology 248:1-25 (Lo, ed., Human Press, Totowa, NJ, 2003). Indeed, naturally occurring camelid antibodies consisting of a heavy chain only are functional and stable in the absence of light chain. See, *e.g.,* Hamers-Casterman et al., Nature 363:446-448 (1993); Sheriff et al., Nature Struct. Biol. 3:733-736 (1996).

A number of HVR delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, MD. (1991)). Chothia refers instead to the location of the structural loops (Chothia and Lesk, J. Mol. Biol. 196:901-917 (1987)). The AbM HVRs represent a compromise between the Kabat HVRs and Chothia structural loops, and are used by Oxford Molecular's AbM antibody modeling software. The "contact" HVRs are based on an analysis of the available complex crystal structures. The residues from each of these HVRs are noted below.

| Loop | Kabat | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B (Kabat numbering) |
| H1 | H31-H35 | H26-H35 | H26-H32 | H30-H35 (Chothia numbering) |
| H2 | H50-H65 | H50-H58 | H53-H55 | H47-H58 |
| H3 | H95-H102 | H95-H102 | H96-H101 | H93-H101 |

HVRs may comprise "extended HVRs" as follows: 24-36 or 24-34 (L1), 46-56 or 50-56 (L2) and 89-97 or 89-96 (L3) in the VL and 26-35 (H1), 50-65 or 49-65 (H2) and 93-102, 94-102, or 95-102 (H3) in the VH. The variable domain residues are numbered according to Kabat *et al., supra*, for each of these definitions.

The expression "variable-domain residue-numbering as in Kabat" or "amino-acid-position numbering as in Kabat," and variations thereof, refers to the numbering system used for heavy-chain variable domains or light-chain variable domains of the compilation of antibodies in Kabat *et al*., *supra*. Using this numbering system, the actual linear amino acid sequence may contain fewer or additional amino acids corresponding to a shortening of, or insertion into, a FR or HVR of the variable domain. For example, a heavy-chain variable domain may include a single amino acid insert (residue 52a according to Kabat) after residue 52 of H2 and inserted residues (*e.g.* residues 82a, 82b, and 82c, *etc*. according to Kabat) after heavy-chain FR residue 82. The Kabat numbering of residues may be determined for a given antibody by alignment at regions of homology of the sequence of the antibody with a "standard" Kabat numbered sequence.

"Framework" or "FR" residues are those variable-domain residues other than the HVR residues as herein defined.

A "human consensus framework" or "acceptor human framework" is a framework that represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, 5th Ed. Public Health Service, National Institutes of Health, Bethesda, MD (1991)*.* Examples include for the VL, the subgroup may be subgroup kappa I, kappa II, kappa III or kappa IV as in Kabat *et al., supra.* Additionally, for the VH, the subgroup may be subgroup I, subgroup II, or subgroup III as in Kabat *et al*.*, supra*. Alternatively, a human consensus framework can be derived from the above in which particular residues, such as when a human framework residue is selected based on its homology to the donor framework by aligning the donor framework sequence with a collection of various human framework sequences. An acceptor human framework "derived from" a human immunoglobulin framework or a human consensus framework may comprise the same amino acid sequence thereof, or it may contain pre-existing amino acid sequence changes. In some embodiments, the number of pre-existing amino acid changes are 10 or less, 9 or less, 8 or less, 7 or less, 6 or less, 5 or less, 4 or less, 3 or less, or 2 or less.

A "VH subgroup III consensus framework" comprises the consensus sequence obtained from the amino acid sequences in variable heavy subgroup III of Kabat *et al., supra.* In one embodiment, the VH subgroup III consensus framework amino acid sequence comprises at least a portion or all of each of the following sequences: EVQLVESGGGLVQPGGSLRLSCAAS (HC-FR1)(SEQ ID NO:35), WVRQAPGKGLEWV (HC-FR2), (SEQ ID NO:36),
RFTISADTSKNTAYLQMNSLRAEDTAVYYCAR (HC-FR3, SEQ ID NO:37),
WGQGTLVTVSA (HC-FR4), (SEQ ID NO:38).

A "VL kappa I consensus framework" comprises the consensus sequence obtained from the amino acid sequences in variable light kappa subgroup I of Kabat *et al., supra.* In one embodiment, the VH subgroup I consensus framework amino acid sequence comprises at least a portion or all of each of the following sequences: DIQMTQSPSSLSASVGDRVTITC (LC-FR1) (SEQ ID NO:39), WYQQKPGKAPKLLIY (LC-FR2) (SEQ ID NO:40),
GVPSRFSGSGSGTDFTLTISSLQPEDFATYYC (LC-FR3)(SEQ ID NO:41), FGQGTKVEIKR (LC-FR4)(SEQ ID NO:42).

An "amino-acid modification" at a specified position, *e.g*. of the Fc region, refers to the substitution or deletion of the specified residue, or the insertion of at least one amino acid residue adjacent the specified residue. Insertion "adjacent" to a specified residue means insertion within one to two residues thereof. The insertion may be N-terminal or C-terminal to the specified residue. The preferred amino acid modification herein is a substitution.

An "affinity-matured" antibody is one with one or more alterations in one or more HVRs thereof that result in an improvement in the affinity of the antibody for antigen, compared to a parent antibody that does not possess those alteration(s). In one embodiment, an affinity-matured antibody has nanomolar or even picomolar affinities for the target antigen. Affinity-matured antibodies are produced by procedures known in the art. For example, Marks et al., Bio/Technology 10:779-783 (1992) describes affinity maturation by VH- and VL-domain shuffling. Random mutagenesis of HVR and/or framework residues is described by, for example: Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

As use herein, the term "specifically binds to" or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. In one embodiment, the extent of binding of an antibody to an unrelated target is less than about 10% of the binding of the antibody to the target as measured, *e.g.,* by a radioimmunoassay (RIA). In certain embodiments, an antibody that specifically binds to a target has a dissociation constant (Kd) of ≤ 1µM, ≤ 100 nM, ≤ 10 nM, ≤ 1 nM, or ≤ 0.1 nM. In certain embodiments, an antibody specifically binds to an epitope on a protein that is conserved among the protein from different species. In another embodiment, specific binding can include, but does not require exclusive binding.

The term "Fc region" herein is used to define a C-terminal region of an immunoglobulin heavy chain, including native-sequence Fc regions and variant Fc regions. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. Suitable native-sequence Fc regions for use in the antibodies of the invention include human IgG1, IgG2 (IgG2A, IgG2B), IgG3 and IgG4.

"Fc receptor" or "FcR" describes a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors, FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see M. Daëron, Annu. Rev. Immunol. 15:203-234 (1997). FcRs are reviewed in Ravetch and Kinet, Annu. Rev. Immunol. 9: 457-92 (1991); Capel et al., Immunomethods 4: 25-34 (1994); and de Haas et al., J. Lab. Clin. Med. 126: 330-41 (1995). Other FcRs, including those to be identified in the future, are encompassed by the term "FcR" herein.

The term "Fc receptor" or "FcR" also includes the neonatal receptor, *FcRn,* which is responsible for the transfer of maternal IgGs to the fetus. Guyer et al., J. Immunol. 117: 587 (1976) and Kim et al., J. Immunol. 24: 249 (1994). Methods of measuring binding to FcRn are known (see, *e.g.,* Ghetie and Ward, Immunol. Today 18: (12): 592-8 (1997); Ghetie et al., Nature Biotechnology 15 (7): 637-40 (1997); Hinton et al., J. Biol. Chem. 279 (8): 6213-6 (2004); WO 2004/92219 (Hinton et al.). Binding to FcRn in vivo and serum half-life of human FcRn high-affinity binding polypeptides can be assayed, *e.g.,* in transgenic mice or transfected human cell lines expressing human FcRn, or in primates to which the polypeptides having a variant Fc region are administered. WO 2004/42072 (Presta) describes antibody variants which improved or diminished binding to FcRs. See also, *e.g.,* Shields et al., J. Biol. Chem. 9(2): 6591-6604 (2001).

The phrase "substantially reduced," or "substantially different," as used herein, denotes a sufficiently high degree of difference between two numeric values (generally one associated with a molecule and the other associated with a reference/comparator molecule) such that one of skill in the art would consider the difference between the two values to be of statistical significance within the context of the biological characteristic measured by said values (*e.g.,* Kd values). The difference between said two values is, for example, greater than about 10%, greater than about 20%, greater than about 30%, greater than about 40%, and/or greater than about 50% as a function of the value for the reference/comparator molecule.

The term "substantially similar" or "substantially the same," as used herein, denotes a sufficiently high degree of similarity between two numeric values (for example, one associated with an antibody of the invention and the other associated with a reference/comparator antibody), such that one of skill in the art would consider the difference between the two values to be of little or no biological and/or statistical significance within the context of the biological characteristic measured by said values (*e.g.*, Kd values). The difference between said two values is, for example, less than about 50%, less than about 40%, less than about 30%, less than about 20%, and/or less than about 10% as a function of the reference/comparator value.

"Carriers" as used herein include pharmaceutically acceptable carriers, excipients, or stabilizers that are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN^{™}, polyethylene glycol (PEG), and PLURONICS^{™}.

A "package insert" refers to instructions customarily included in commercial packages of medicaments that contain information about the indications customarily included in commercial packages of medicaments that contain information about the indications, usage, dosage, administration, contraindications, other medicaments to be combined with the packaged product, and/or warnings concerning the use of such medicaments, *etc*.

As used herein, the term "treatment" refers to clinical intervention designed to alter the natural course of the individual or cell being treated during the course of clinical pathology. Desirable effects of treatment include, but are not limited to, decreasing the rate of disease progression, ameliorating or palliating the disease state, remission or improved prognosis, and delaying disease progression. Delaying progression of a disease (*e.g.*, INS) means to defer, hinder, slow, retard, stabilize, and/or postpone development of the disease. This delay can be of varying lengths of time, depending on the history of the disease and/or individual being treated. As is evident to one skilled in the art, a sufficient or significant delay can, in effect, encompass prevention, in that the individual, *e.g.,* an individual at risk for developing the disease, does not develop the disease.

As used herein, "sustained complete remission" refers to a response to treatment that includes a first morning void UPCR ≤0.2g/g without occurrence of relapse or any of certain intercurrent events (*e.g.*, occurring after Week 8) such as (1) relapse as defined by any of the following events requiring systemic corticosteroid or other immunosuppressive treatment: (a) first morning void UPCR≥2 g/g or (b) dipstick UA ≥3+ for 3 consecutive days and the most recent urine sample of the 3-day period determined to have UPCR > 0.2 g/g or (c) dipstick UA protein ≥3+ on any single day with edema and urine sample determined to have UPCR > 0.2g/g; (2) any systemic corticosteroid usage for >14 days in a 30-day period; (3) initiation of any rescue therapy for INS other than systemic corticosteroids; (4) treatment discontinuation due to lack of efficacy; or (5) death.

"CD20" as used herein refers to the human B-lymphocyte antigen CD20 (also known as CD20, B-lymphocyte surface antigen B1, Leu-16, Bp35, BM5, and LF5; the sequence is characterized by the SwissProt database entry P11836) is a hydrophobic transmembrane protein with a molecular weight of approximately 35 kD located on pre-B and mature B lymphocytes. (Valentine, M.A., et al., J. Biol. Chem. 264(19) (1989 11282-11287; Tedder, T.F., et al, Proc. Natl. Acad. Sci. U.S.A. 85 (1988) 208-12; Stamenkovic, I., et al., J. Exp. Med. 167 (1988) 1975-80; Einfeld, D.A., et al., EMBO J. 7 (1988) 711-7; Tedder, T.F., et al., J. Immunol. 142 (1989) 2560-8). The corresponding human gene is Membrane-spanning 4-domains, subfamily A, member 1, also known as MS4A1. This gene encodes a member of the membrane-spanning 4A gene family. Members of this nascent protein family are characterized by common structural features and similar intron/exon splice boundaries and display unique expression patterns among hematopoietic cells and nonlymphoid tissues. This gene encodes the B-lymphocyte surface molecule which plays a role in the development and differentiation of B-cells into plasma cells. This family member is localized to 11q12, among a cluster of family members. Alternative splicing of this gene results in two transcript variants which encode the same protein.

The terms "CD20" and "CD20 antigen" are used interchangeably herein, and include any variants, isoforms and species homologs of human CD20 which are naturally expressed by cells or are expressed on cells transfected with the CD20 gene. Binding of an antibody of the invention to the CD20 antigen mediate the killing of cells expressing CD20 (e.g., a tumor cell) by inactivating CD20. The killing of the cells expressing CD20 may occur by one or more of the following mechanisms: Cell death/apoptosis induction, ADCC and CDC.

Synonyms of CD20, as recognized in the art, include B-lymphocyte antigen CD20, B-lymphocyte surface antigen B1, Leu-16, Bp35, BM5, and LF5.

The term "anti-CD20 antibody" according to the invention is an antibody that binds specifically to CD20 antigen. Depending on binding properties and biological activities of anti-CD20 antibodies to the CD20 antigen, two types of anti-CD20 antibodies (type I and type II anti-CD20 antibodies) can be distinguished according to Cragg, M.S., et al., Blood 103 (2004) 2738-2743; and Cragg, M.S., et al., Blood 101 (2003) 1045-1052, see Table 1 below.

**Table 1. Properties of type I and type II anti-CD20 antibodies**

| **Type I anti-CD20 antibodies** | **type II anti-CD20 antibodies** |
|---|---|
| type I CD20 epitope | type II CD20 epitope |
| Localize CD20 to lipid rafts | Do not localize CD20 to lipid rafts |
| Increased CDC (if IgG1 isotype) | Decreased CDC (if IgG1 isotype) |
| ADCC activity (if IgG1 isotype) | ADCC activity (if IgG1 isotype) |
| Full binding capacity | Reduced binding capacity |
| Homotypic aggregation | Stronger homotypic aggregation |
| Apoptosis induction upon cross-linking | Strong cell death induction without cross-linking |

Examples of type II anti-CD20 antibodies include e.g. humanized B-Ly1 antibody IgG1 (a chimeric humanized IgG1 antibody as disclosed in WO 2005/044859), 11B8 IgG1 (as disclosed in WO 2004/035607), and AT80 IgG1. Typically type II anti-CD20 antibodies of the IgG1 isotype show characteristic CDC properties. Type II anti-CD20 antibodies have a decreased CDC (if IgG1 isotype) compared to type I antibodies of the IgG1 isotype.

Examples of type I anti-CD20 antibodies include e.g. rituximab, HI47 IgG3 (ECACC, hybridoma), 2C6 IgG1 (as disclosed in WO 2005/103081), 2F2 IgG1 (as disclosed and WO 2004/035607 and WO 2005/103081) and 2H7 IgG1 (as disclosed in WO 2004/056312).

The afucosylated anti-CD20 antibodies according to the invention are preferably type II anti-CD20 antibodies, more preferably afucosylated humanized B-Ly1 antibodies as described in WO 2005/044859 and WO 2007/031875.

The "rituximab" antibody (reference antibody; example of a type I anti-CD20 antibody) is a genetically engineered chimeric human gamma 1 murine constant domain containing monoclonal antibody directed against the human CD20 antigen. However this antibody is not glycoengineered and not afocusylates and thus has an amount of fucose of at least 85 %. This chimeric antibody contains human gamma 1 constant domains and is identified by the name "C2B8" in US 5,736,137 (Andersen, et. al.) issued on April 17, 1998, assigned to IDEC Pharmaceuticals Corporation. Rituximab is approved for the treatment of patients with relapsed or refracting low-grade or follicular, CD20 positive, B cell non-Hodgkin's lymphoma. In vitro mechanism of action studies have shown that rituximab exhibits human complement-dependent cytotoxicity (CDC) (Reff, M.E., et. al, Blood 83(2) (1994) 435-445). Additionally, it exhibits activity in assays that measure antibody-dependent cellular cytotoxicity (ADCC).

The term "GA101 antibody" as used herein refers to any one of the following antibodies that bind human CD20: (1) an antibody comprising an HVR-H1 comprising the amino acid sequence of SEQ ID NO:1, an HVR-H2 comprising the amino acid sequence of SEQ ID NO:2, an HVR-H3 comprising the amino acid sequence of SEQ ID NO:3, an HVR-L1 comprising the amino acid sequence of SEQ ID NO:4, an HVR-L2 comprising the amino acid sequence of SEQ ID NO:5, and an HVR-L3 comprising the amino acid sequence of SEQ ID NO:6; (2) an antibody comprising a VH domain comprising the amino acid sequence of SEQ ID NO:7 and a VL domain comprising the amino acid sequence of SEQ ID NO:8, (3) an antibody comprising an amino acid sequence of SEQ ID NO:9 and an amino acid sequence of SEQ ID NO: 10; (4) an antibody known as obinutuzumab, or (5) an antibody that comprises an amino acid sequence that has at least 95%, 96%, 97%, 98% or 99% sequence identity with amino acid sequence of SEQ ID NO:9 and that comprises an amino acid sequence that has at least 95%, 96%, 97%, 98% or 99% sequence identity with an amino acid sequence of SEQ ID NO:10. In one embodiment, the GA101 antibody is an IgG1 isotype antibody. In some embodiments, the anti-CD20 antibody is a humanized B-Ly1 antibody.

The term "humanized B-Ly1 antibody" refers to humanized B-Ly1 antibody as disclosed in WO 2005/044859 and WO 2007/031875, which were obtained from the murine monoclonal anti-CD20 antibody B-Ly1 (variable region of the murine heavy chain (VH): SEQ ID NO: 11; variable region of the murine light chain (VL): SEQ ID NO: 12- see Poppema, S. and Visser, L., Biotest Bulletin 3 (1987) 131-139) by chimerization with a human constant domain from IgG1 and following humanization (see WO 2005/044859 and WO 2007/031875). These "humanized B-Ly1 antibodies" are disclosed in detail in WO 2005/ 044859 and WO 2007/031875.
Variable region of the murine monoclonal anti-CD20 antibody B-Ly1 heavy chain (VH) (SEQ ID NO: 11) 
Gly Pro Glu Leu Val Lys Pro Gly Ala Ser Val Lys Ile Ser Cys Lys
1               5                   10                  15
Ala Ser Gly Tyr Ala Phe Ser Tyr Ser Trp Met Asn Trp Val Lys Leu
            20                  25                  30
Arg Pro Gly Gln Gly Leu Glu Trp Ile Gly Arg Ile Phe Pro Gly Asp
        35                  40                  45
Gly Asp Thr Asp Tyr Asn Gly Lys Phe Lys Gly Lys Ala Thr Leu Thr
    50                  55                  60
Ala Asp Lys Ser Ser Asn Thr Ala Tyr Met Gln Leu Thr Ser Leu Thr
65                  70                  75                  80
Ser Val Asp Ser Ala Val Tyr Leu Cys Ala Arg Asn Val Phe Asp Gly
                85                  90                  95
Tyr Trp Leu Val Tyr Trp Gly Gln Gly Thr Leu Val Thr Val Ser Ala
            100                 105                 110
Variable region of the murine monoclonal anti-CD20 antibody B-Ly1 light chain (VL) (SEQ ID NO: 12)

Asn Pro Val Thr Leu Gly Thr Ser Ala Ser Ile Ser Cys Arg Ser Ser
1               5                   10                  15
Lys Ser Leu Leu His Ser Asn Gly Ile Thr Tyr Leu Tyr Trp Tyr Leu
            20                  25                  30
Gln Lys Pro Gly Gln Ser Pro Gln Leu Leu Ile Tyr Gln Met Ser Asn
        35                  40                  45
Leu Val Ser Gly Val Pro Asp Arg Phe Ser Ser Ser Gly Ser Gly Thr
    50                  55                  60
Asp Phe Thr Leu Arg Ile Ser Arg Val Glu Ala Glu Asp Val Gly Val
65                  70                  75                  80
Tyr Tyr Cys Ala Gln Asn Leu Glu Leu Pro Tyr Thr Phe Gly Gly Gly
                85                  90                  95
Thr Lys Leu Glu Ile Lys Arg
            100

In one embodiment, the "humanized B-Ly1 antibody" has variable region of the heavy chain (VH) selected from group of SEQ ID NO:7, 8, and 13 to 33 (corresponding to, inter alia, B-HH2 to B-HH9 and B-HL8 to B-HL17 of WO 2005/044859 and WO 2007/031875). In one specific embodiment, such variable domain is selected from the group consisting of SEQ ID NOS:14, 15, 7, 19, 25, 27, and 29 (corresponding to B-HH2, BHH-3, B-HH6, B-HH8, B-HL8, B-HL11 and B-HL13 of WO 2005/044859 and WO 2007/031875). In one specific embodiment, the "humanized B-Ly1 antibody" has variable region of the light chain (VL) of SEQ ID NO:8 (corresponding to B-KV1 of WO 2005/044859 and WO 2007/031875). In one specific embodiment, the "humanized B-Ly1 antibody" has a variable region of the heavy chain (VH) of SEQ ID NO:7 (corresponding to B-HH6 of WO 2005/044859 and WO 2007/031875) and a variable region of the light chain (VL) of SEQ ID NO:8 (corresponding to B-KV1 of WO 2005/044859 and WO 2007/031875). Furthermore in one embodiment, the humanized B-Ly1 antibody is an IgG1 antibody. According to the invention such afocusylated humanized B-Ly1 antibodies are glycoengineered (GE) in the Fc region according to the procedures described in WO 2005/044859, WO 2004/065540, WO 2007/031875, Umana, P. et al., Nature Biotechnol. 17 (1999) 176-180 and WO 99/154342. In one embodiment, the afucosylated glyco-engineered humanized B-Ly1 is B-HH6-B-KV1 GE. In one embodiment, the anti-CD20 antibody is obinutuzumab (recommended INN, WHO Drug Information, Vol. 26, No. 4, 2012, p. 453). As used herein, obinutuzumab is synonymous for GA101 or RO5072759. This replaces all previous versions (e.g. Vol. 25, No. 1, 2011, p.75-76), and is formerly known as afutuzumab (recommended INN, WHO Drug Information, Vol. 23, No. 2, 2009, p. 176;Vol. 22, No. 2, 2008, p. 124). As used herein, references to obinutuzumab refer to GAZYVA^{®} as well as biosimilar antibodies thereof. In some embodiments, the humanized B-Ly1 antibody is an antibody comprising a heavy chain comprising the amino acid sequence of SEQ ID NO:9 and a light chain comprising the amino acid sequence of SEQ ID NO:10 or an antigen-binding fragment thereof. In some embodiments, the humanized B-Ly1 antibody comprises a heavy chain variable region comprising the three heavy chain CDRs of SEQ ID NO:9 and a light chain variable region comprising the three light chain CDRs of SEQ ID NO:10.
Heavy chain (SEQ ID NO:9) 
QVQLVQSGAE VKKPGSSVKV SCKASGYAFS YSWINWVRQA PGQGLEWMGR 50
IFPGDGDTDY NGKFKGRVTI TADKSTSTAY MELSSLRSED TAVYYCARNV 100
FDGYWLVYWG QGTLVTVSSA STKGPSVFPL APSSKSTSGG TAALGCLVKD 150
YFPEPVTVSW NSGALTSGVH TFPAVLQSSG LYSLSSVVTV PSSSLGTQTY 200
ICNVNHKPSN TKVDKKVEPK SCDKTHTCPP CPAPELLGGP SVFLFPPKPK 250
DTLMISRTPE VTCVVVDVSH EDPEVKFNWY VDGVEVHNAK TKPREEQYNS 300
TYRVVSVLTV LHQDWLNGKE YKCKVSNKAL PAPIEKTISK AKGQPREPQV 350
YTLPPSRDEL TKNQVSLTCL VKGFYPSDIA VEWESNGQPE NNYKTTPPVL 400
DSDGSFFLYS KLTVDKSRWQ QGNVFSCSVM HEALHNHYTQ KSLSLSPG 449
Light chain (SEQ ID NO:10)

DIVMTQTPLS LPVTPGEPAS ISCRSSKSLL HSNGITYLYW YLQKPGQSPQ 50

LLIYQMSNLV SGVPDRFSGS GSGTDFTLKI SRVEAEDVGV YYCAQNLELP 100
YTFGGGTKVE IKRTVAAPSV FIFPPSDEQL KSGTASVVCL LNNFYPREAK 150
VQWKVDNALQ SGNSQESVTE QDSKDSTYSL SSTLTLSKAD YEKHKVYACE 200
VTHQGLSSPV TKSFNRGEC 219

In some embodiments, the humanized B-Ly1 antibody is an afucosylated glyco-engineered humanized B-Ly1. Such glycoengineered humanized B-Ly1 antibodies have an altered pattern of glycosylation in the Fc region, preferably having a reduced level of fucose residues. Preferably the amount of fucose is 60 % or less of the total amount of oligosaccharides at Asn297 (in one embodiment the amount of fucose is between 40 % and 60 %, in another embodiment the amount of fucose is 50 % or less, and in still another embodiment the amount of fucose is 30 % or less). Furthermore the oligosaccharides of the Fc region are preferably bisected. These glycoengineered humanized B-Ly1 antibodies have an increased ADCC.

The "ratio of the binding capacities to CD20 on Raji cells (ATCC-No. CCL-86) of an anti-CD20 antibodies compared to rituximab" is determined by direct immunofluorescence measurement (the mean fluorescence intensities (MFI) is measured) using said anti-CD20 antibody conjugated with Cy5 and rituximab conjugated with Cy5 in a FACSArray (Becton Dickinson) with Raji cells (ATCC-No. CCL-86), as described in Example No. 2, and calculated as follows: $\begin{matrix}Ratio of the binding capacities to CD 20 on Raji cells \left(ATTC-No. CCL-86\right) = \\ \frac{MFI \left(Cy5-anti-CD20 antibody\right)}{MFI \left(Cy5-rituximab\right)} \times \frac{Cy 5 - labeling ratio \left(Cy5-rituximab\right)}{Cy 5 - labeling ratio \left(Cy5-anti-CD20 antibody\right)}\end{matrix}$

MFI is the mean fluorescent intensity. The "Cy5-labeling ratio" as used herein means the number of Cy5-label molecules per molecule antibody.

Typically said type II anti-CD20 antibody has a ratio of the binding capacities to CD20 on Raji cells (ATCC-No. CCL-86) of said second anti-CD20 antibody compared to rituximab of 0.3 to 0.6, and in one embodiment, 0.35 to 0.55, and in yet another embodiment, 0.4 to 0.5.

In one embodiment said type II anti-CD20 antibody, e.g., a GA101 antibody, has increased antibody dependent cellular cytotoxicity (ADCC).

By "antibody having increased antibody dependent cellular cytotoxicity (ADCC)", it is meant an antibody, as that term is defined herein, having increased ADCC as determined by any suitable method known to those of ordinary skill in the art. One accepted in vitro ADCC assay is as follows:
1) the assay uses target cells that are known to express the target antigen recognized by the antigen-binding region of the antibody;
2) the assay uses human peripheral blood mononuclear cells (PBMCs), isolated from blood of a randomly chosen healthy donor, as effector cells;
3) the assay is carried out according to following protocol:
   i) the PBMCs are isolated using standard density centrifugation procedures and are suspended at 5 x 10⁶ cells/ml in RPMI cell culture medium;
   ii) the target cells are grown by standard tissue culture methods, harvested from the exponential growth phase with a viability higher than 90%, washed in RPMI cell culture medium, labeled with 100 micro-Curies of ⁵¹Cr, washed twice with cell culture medium, and resuspended in cell culture medium at a density of 10⁵ cells/ml;
   iii) 100 microliters of the final target cell suspension above are transferred to each well of a 96-well microtiter plate;
   iv) the antibody is serially-diluted from 4000 ng/ml to 0.04 ng/ml in cell culture medium and 50 microliters of the resulting antibody solutions are added to the target cells in the 96-well microtiter plate, testing in triplicate various antibody concentrations covering the whole concentration range above;
   v) for the maximum release (MR) controls, 3 additional wells in the plate containing the labeled target cells, receive 50 microliters of a 2% (VN) aqueous solution of non-ionic detergent (Nonidet, Sigma, St. Louis), instead of the antibody solution (point iv above);
   vi) for the spontaneous release (SR) controls, 3 additional wells in the plate containing the labeled target cells, receive 50 microliters of RPMI cell culture medium instead of the antibody solution (point iv above);
   vii) the 96-well microtiter plate is then centrifuged at 50 x g for 1 minute and incubated for 1 hour at 4°C;
   viii) 50 microliters of the PBMC suspension (point i above) are added to each well to yield an effector:target cell ratio of 25:1 and the plates are placed in an incubator under 5% CO2 atmosphere at 37°C for 4 hours;
   ix) the cell-free supernatant from each well is harvested and the experimentally released radioactivity (ER) is quantified using a gamma counter;
   x) the percentage of specific lysis is calculated for each antibody concentration according to the formula (ER-MR)/(MR-SR) x 100, where ER is the average radioactivity quantified (see point ix above) for that antibody concentration, MR is the average radioactivity quantified (see point ix above) for the MR controls (see point V above), and SR is the average radioactivity quantified (see point ix above) for the SR controls (see point vi above);
4) "increased ADCC" is defined as either an increase in the maximum percentage of specific lysis observed within the antibody concentration range tested above, and/or a reduction in the concentration of antibody required to achieve one half of the maximum percentage of specific lysis observed within the antibody concentration range tested above. In one embodiment, the increase in ADCC is relative to the ADCC, measured with the above assay, mediated by the same antibody, produced by the same type of host cells, using the same standard production, purification, formulation and storage methods, which are known to those skilled in the art, except that the comparator antibody (lacking increased ADCC) has not been produced by host cells engineered to overexpress GnTIII and/or engineered to have reduced expression from the fucosyltransferase 8 (FUT8) gene (e.g., including, engineered for FUT8 knock out).

Said "increased ADCC" can be obtained by, for example, mutating and/or glycoengineering of said antibodies. In one embodiment, the antibody is glycoengineered to have a biantennary oligosaccharide attached to the Fc region of the antibody that is bisected by GlcNAc, e.g., in WO 2003/011878 (Jean-Mairet et al.); US Patent No. 6,602,684 (Umana et al.); US 2005/0123546 (Umana et al.), Umana, P., et al., Nature Biotechnol. 17 (1999) 176-180). In another embodiment, the antibody is glycoengineered to lack fucose on the carbohydrate attached to the Fc region by expressing the antibody in a host cell that is deficient in protein fucosylation (e.g., Lec13 CHO cells or cells having an alpha-1,6-fucosyltransferase gene (FUT8) deleted or the FUT gene expression knocked down (see, e.g., Yamane-Ohnuki et al. Biotech. Bioeng. 87: 614 (2004); Kanda, Y. et al., Biotechnol. Bioeng., 94(4):680-688 (2006); and WO2003/085107). In yet another embodiment, the antibody sequence has been engineered in its Fc region to enhance ADCC (e.g., in one embodiment, such engineered antibody variant comprises an Fc region with one or more amino acid substitutions at positions 298, 333, and/or 334 of the Fc region (EU numbering of residues)).

The term "complement-dependent cytotoxicity (CDC)" refers to lysis of human tumor target cells by the antibody according to the invention in the presence of complement. CDC can be measured by the treatment of a preparation of CD20 expressing cells with an anti-CD20 antibody according to the invention in the presence of complement. CDC is found if the antibody induces at a concentration of 100 nM the lysis (cell death) of 20% or more of the tumor cells after 4 hours. In one embodiment, the assay is performed with ⁵¹Cr or Eu labeled tumor cells and measurement of released ⁵¹Cr or Eu. Controls include the incubation of the tumor target cells with complement but without the antibody.

The term "expression of the CD20" antigen is intended to indicate a significant level of expression of the CD20 antigen in a cell, e.g., a T- or B- Cell. In one embodiment, patients to be treated according to the methods of this invention express significant levels of CD20 on a B-cell. CD20 expression on a B-cell can be determined by standard assays known in the art. e.g., CD20 antigen expression is measured using immunohistochemical (IHC) detection, FACS or via PCR-based detection of the corresponding mRNA.

As used in this specification and the appended claims, the singular forms "a", "an" and "the" include plural referents unless the content clearly dictates otherwise. Thus, for example, reference to "a molecule" optionally includes a combination of two or more such molecules, and the like.

The term "about" as used herein refers to the usual error range for the respective value readily known to the skilled person in this technical field. Reference to "about" a value or parameter herein includes (and describes) embodiments that are directed to that value or parameter per se.

It is understood that aspects and embodiments of the invention described herein include "comprising," "consisting," and "consisting essentially of" aspects and embodiments.

### III. Methods

In one aspect, provided herein are methods for treating childhood-onset idiopathic nephrotic syndrome (INS) in an individual by administering an effective amount of a type II anti-CD20 antibody; wherein the individual is a human that is greater than or equal to 2 years of age and less than or equal to 25 years of age. In another aspect, provided herein are methods for reducing risk and/or frequency of relapse in an individual with childhood-onset idiopathic nephrotic syndrome (INS) by administering an effective amount of a type II anti-CD20 antibody; wherein the individual is a human that is greater than or equal to 2 years of age and less than or equal to 25 years of age. In one aspect, provided herein are methods for treating childhood-onset INS in an individual or depleting circular peripheral B cells in an individual with childhood-onset INS by administering an effective amount of a type II anti-CD20 antibody; wherein the individual is a human that is greater than or equal to 2 years of age and less than or equal to 25 years of age.

In some embodiments, *e.g.*, when the individual weighs greater than or equal to 45kg, the methods include administering to the individual a first antibody exposure to a type II anti-CD20 antibody and a second antibody exposure to the type II anti-CD20 antibody, the second antibody exposure not being provided until from about 18 weeks to about 26 weeks after the first antibody exposure; wherein the first antibody exposure comprises one or two doses of the type II anti-CD20 antibody, the first antibody exposure comprising a total exposure of between about 1800mg and about 2200mg of the type II anti-CD20 antibody; and wherein the second antibody exposure comprises one or two doses of the type II anti-CD20 antibody, the second antibody exposure comprising a total exposure of between about 1800mg and about 2200mg of the type II anti-CD20 antibody. In some embodiments, *e.g.*, when the individual weighs less than 45kg, the methods include administering to the individual a first antibody exposure to a type II anti-CD20 antibody and a second antibody exposure to the type II anti-CD20 antibody, the second antibody exposure not being provided until from about 18 weeks to about 26 weeks after the first antibody exposure, wherein the first antibody exposure comprises one or two doses of the type II anti-CD20 antibody, the first antibody exposure comprising a total exposure of between about 36mg/kg and about 44mg/kg of the type II anti-CD20 antibody; wherein the second antibody exposure comprises one or two doses of the type II anti-CD20 antibody, the second antibody exposure comprising a total exposure of between about 36mg/kg and about 44mg/kg of the type II anti-CD20 antibody. As described herein, in some embodiments, the antibody comprises a heavy chain comprising HVR-H1 sequence of SEQ ID NO:1, HVR-H2 sequence of SEQ ID NO:2, and HVR-H3 sequence of SEQ ID NO:3, and a light chain comprising HVR-L1 sequence of SEQ ID NO:4, HVR-L2 sequence of SEQ ID NO:5, and HVR-L3 sequence of SEQ ID NO:6. In some embodiments, the antibody comprises a VH domain comprising the amino acid sequence of SEQ ID NO:7 and a VL domain comprising the amino acid sequence of SEQ ID NO:8. In some embodiments, the antibody comprises an amino acid sequence of SEQ ID NO:9 and an amino acid sequence of SEQ ID NO:10. In some embodiments, the antibody comprises an antibody that comprises an amino acid sequence that has at least 95%, 96%, 97%, 98% or 99% sequence identity with amino acid sequence of SEQ ID NO:9 and that comprises an amino acid sequence that has at least 95%, 96%, 97%, 98% or 99% sequence identity with an amino acid sequence of SEQ ID NO:10. In some embodiments, the antibody is obinutuzumab.

### Anti-CD20 antibodies

Certain aspects of the present disclosure relate to anti-CD20 antibodies, *e.g.,* for use in methods described herein, e.g., for treating or reducing risk and/or frequency of relapse of childhood onset INS (*e.g.,* FRNS or SDNS). In some embodiments, the anti-CD20 antibody is a type II antibody. In some embodiments, the anti-CD20 antibody is human or humanized. In some embodiments, the anti-CD20 antibody is afucosylated. In some embodiments, the anti-CD20 antibody is a GA101 antibody.

Examples of type II anti-CD20 antibodies include e.g. humanized B-Ly1 antibody IgG1 (a chimeric humanized IgG1 antibody as disclosed in WO 2005/044859), 11B8 IgG1 (as disclosed in WO 2004/035607), and AT80 IgG1. Typically type II anti-CD20 antibodies of the IgG1 isotype show characteristic CDC properties. Type II anti-CD20 antibodies have a decreased CDC (if IgG1 isotype) compared to type I antibodies of the IgG1 isotype.

In some embodiments, the anti-CD20 antibody is a GA101 antibody described herein. In some embodiments, the anti-CD20 is any one of the following antibodies that bind human CD20: (1) an antibody comprising an HVR-H1 comprising the amino acid sequence of GYAFSY (SEQ ID NO:1), an HVR-H2 comprising the amino acid sequence of FPGDGDTD (SEQ ID NO:2), an HVR-H3 comprising the amino acid sequence of NVFDGYWLVY (SEQ ID NO:3), an HVR-L1 comprising the amino acid sequence of RSSKSLLHSNGITYLY (SEQ ID NO:4), an HVR-L2 comprising the amino acid sequence of QMSNLVS (SEQ ID NO:5), and an HVR-L3 comprising the amino acid sequence of AQNLELPYT (SEQ ID NO:6); (2) an antibody comprising a VH domain comprising the amino acid sequence of SEQ ID NO:7 and a VL domain comprising the amino acid sequence of SEQ ID NO:8, (3) an antibody comprising an amino acid sequence of SEQ ID NO:9 and an amino acid sequence of SEQ ID NO:10; (4) an antibody known as obinutuzumab, or (5) an antibody that comprises an amino acid sequence that has at least 95%, 96%, 97%, 98% or 99% sequence identity with amino acid sequence of SEQ ID NO:9 and that comprises an amino acid sequence that has at least 95%, 96%, 97%, 98% or 99% sequence identity with an amino acid sequence of SEQ ID NO:10. In one embodiment, the GA101 antibody is an IgG1 isotype antibody. In some embodiments, the anti-CD20 antibody comprises an HVR-H1, HVR-H2, HVR-H3, HVR-L1, HVR-L2, and HVR-L3 of any of the antibodies described herein, *e.g.,* 3 HVRs from SEQ ID NO:7 and 3 HVRs from SEQ ID NO:8, 3 HVRs from SEQ ID NO:9 and 3 HVRs from SEQ ID NO:10, or any HVRs of the amino acid sequences provided in Table 2.

In some embodiments, the anti-CD20 antibody comprises a heavy chain variable region (VH) comprising the amino acid sequence of SEQ ID NO:7, and a light chain variable region (VL) comprising the amino acid sequence of SEQ ID NO:8.

QVQLVQSGAEVKKPGSSVKVSCKASGYAFSYSWINWVRQAPGQGLEWMGRIFPGDGD
TDYNGKFKGRVTITADKSTSTAYMELSSLRSEDTAVYYCARNVFDGYWLVYWGQGTL
VTVSS (SEQ ID NO:7)

DIVMTQTPLSLPVTPGEPASISCRSSKSLLHSNGITYLYWYLQKPGQSPQLLIYQMSNLV
SGVPDRFSGSGSGTDFTLKISRVEAEDVGVYYCAQNLELPYTFGGGTKVEIKRTV (SEQ
ID NO:8).

In some embodiments, the anti-CD20 antibody comprises a heavy chain comprising the amino acid sequence of SEQ ID NO:9, and a light chain comprising the amino acid sequence of SEQ ID NO:10.

QVQLVQSGAEVKKPGSSVKVSCKASGYAFSYSWINWVRQAPGQGLEWMGRIFPGDGDTDYNGKF
KGRVTITADKSTSTAYMELSSLRSEDTAVYYCARNVFDGYWLVYWGQGTLVTVSSASTKGPSVF
PLAPSSKSTSGGTAALGCLVKDYFPEPVTVSWNSGALTSGVHTFPAVLQSSGLYSLSSVVTVPS
SSLGTQTYICNVNHKPSNTKVDKKVEPKSCDKTHTCPPCPAPELLGGPSVFLFPPKPKDTLMIS
RTPEVTCVVVDVSHEDPEVKFNWYVDGVEVHNAKTKPREEQYNSTYRVVSVLTVLHQDWLNGKE

YKCKVSNKALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNHYTQKSLSLSPG
(SEQ ID NO:9)

DIVMTQTPLSLPVTPGEPASISCRSSKSLLHSNGITYLYWYLQKPGQSPQLLIYQMSNLVSGVP
DRFSGSGSGTDFTLKISRVEAEDVGVYYCAQNLELPYTFGGGTKVEIKRTVAAPSVFIFPPSDE
QLKSGTASVVCLLNNFYPREAKVQWKVDNALQSGNSQESVTEQDSKDSTYSLSSTLTLSKADYE
KHKVYACEVTHQGLSSPVTKSFNRGEC (SEQ ID NO:10)

In some embodiments, the anti-CD20 antibody is a humanized B-Ly1 antibody. In some embodiments, the humanized B-Ly1 antibody comprises a heavy chain variable region comprising the three heavy chain CDRs of SEQ ID NO:9 and a light chain variable region comprising the three light chain CDRs of SEQ ID NO:10. In some embodiments, the humanized B-Ly1 antibody comprises a heavy chain comprising the sequence of SEQ ID NO:9 and a light chain comprising the sequence of SEQ ID NO:10.

In some embodiments, the anti-CD20 antibody comprises an amino acid sequence at least 80%, 85%, 90%, 95%, 96%, 97%, 98% or 99% identical to a polypeptide sequence listed in Table 2 below.

**Table 2. Polypeptide sequences.**

| **CONSTRUCT** | **POLYPEPTIDE SEQUENCE** | **SEQ ID NO** |
|---|---|---|
| B-HH1 | QVQLVQSGAEVKKPGSSVKVSCKASGYTFSYSWM SWVRQAPGQGLEWMGRIFPGDGDTDYAQKFQGRV TITADKSTSTAYMELSSLRSEDTAVYYCARNVFDG YWLVYWGQGTLVTVSS | 13 |
| B-HH2 | QVQLVQSGAEVKKPGSSVKVSCKASGYAFSYSWM NWVRQAPGQGLEWMGRIFPGDGDTDYNGKFKGR VTITADKSTSTAYMELSSLRSEDTAVYYCARNVFD GYWLVYWGQGTLVTVSS | 14 |
| B-HH3 | QVQLVQSGAEVKKPGSSVKVSCKASGYAFSYSWM NWVRQAPGQGLEWMGRIFPGDGDTDYNGKFKGR VTITADKSTSTAYMELSSLRSEDTAVYLCARNVFDG YWLVYWGQGTLVTVSS | 15 |
| B-HH4 | QVQLVQSGAEVKKPGASVKVSCKVSGYAFSYSWM NWVRQAPGQGLEWMGRIFPGDGDTDYNGKFKGR VTITADKSTSTAYMELSSLRSEDTAVYYCARNVFD GYWLVYWGQGTLVTVSS | 16 |
| B-HH5 | QVQLVQSGAEVKKPGSSVKVSCKASGYAFSYSWM SWVRQAPGQGLEWMGRIFPGDGDTDYNGKFKGRV TITADKSTSTAYMELSSLRSEDTAVYYCARNVFDG YWLVYWGQGTLVTVSS | 17 |
| B-HH6 | QVQLVQSGAEVKKPGSSVKVSCKASGYAFSYSWIN WVRQAPGQGLEWMGRIFPGDGDTDYNGKFKGRVT ITADKSTSTAYMELSSLRSEDTAVYYCARNVFDGY WLVYWGQGTLVTVSS | 7 |
| B-HH7 | QVQLVQSGAEVKKPGSSVKVSCKASGYAFSYSWIS WVRQAPGQGLEWMGRIFPGDGDTDYNGKFKGRVT ITADKSTSTAYMELSSLRSEDTAVYYCARNVFDGY WLVYWGQGTLVTVSS | 18 |
| B-HH8 | QVQLVQSGAEVKKPGASVKVSCKASGYTFTYSWM NWVRQAPGQGLEWMGRIFPGDGDTDYNGKFKGR VTITADKSTSTAYMELSSLRSEDTAVYYCARNVFD GYWLVYWGQGTLVTVSS | 19 |
| B-HH9 | QVQLVQSGAEVKKPGASVKVSCKASGYTFSYSWM NWVRQAPGQGLEWMGRIFPGDGDTDYNGKFKGR VTITADKSTSTAYMELSSLRSEDTAVYYCARNVFD GYWLVYWGQGTLVTVSS | 20 |
| B-HL1 | QVQLVQSGAEVKKPGASVKVSCKASGYTFTYSWM HWVRQAPGQGLEWMGRIFPGDGDTDYAQKFQGR VTMTRDTSTSTVYMELSSLRSEDTAVYYCARNVFD GYWLVYWGQGTLVTVSS | 21 |
| B-HL2 | EVQLVQSGAEVKKPGATVKISCKVSGYTFTYSWMH WVQQAPGKGLEWMGRIFPGDGDTDYAEKFQGRVT ITADTSTDTAYMELSSLRSEDTAVYYCATNVFDGY WLVYWGQGTLVTVSS | 22 |
| B-HL3 | EVQLVQSGAEVKKPGATVKISCKVSGYTFTYSWMN WVQQAPGKGLEWMGRIFPGDGDTDYNGKFKGRVT ITADTSTDTAYMELSSLRSEDTAVYYCATNVFDGY WLVYWGQGTLVTVSS | 23 |
| B-HL4 | QMQLVQSGAEVKKTGSSVKVSCKASGYTFTYSWM SWVRQAPGQGLEWMGRIFPGDGDTDYAQKFQGRV TITADKSTSTAYMELSSLRSEDTAVYYCARNVFDG YWLVYWGQGTLVTVSS | 24 |
| B-HL8 | EVQLVESGGGLVKPGGSLRLSCAASGFTFSYSWMN WVRQAPGKGLEWVGRIFPGDGDTDYNGKFKGRVT ITADKSTSTAYMELSSLRSEDTAVYYCARNVFDGY WLVYWGQGTLVTVSS | 25 |
| B-HL10 | EVQLVESGGGLVKPGGSLRLSCAASGFAFSYSWMN WVRQAPGKGLEWVGRIFPGDGDTDYNGKFKGRVT ITADKSTSTAYMELSSLRSEDTAVYYCARNVFDGY WLVYWGQGTLVTVSS | 26 |
| B-HL11 | QVQLVESGGGLVKPGGSLRLSCAASGFTFSYSWMN WVRQAPGKGLEWVGRIFPGDGDTDYNGKFKGRVT ITADKSTSTAYMELSSLRSEDTAVYYCARNVFDGY WLVYWGQGTLVTVSS | 27 |
| B-HL12 | EVQLVESGAGLVKPGGSLRLSCAASGFTFSYSWMN WVRQAPGKGLEWMGRIFPGDGDTDYNGKFKGRVT ITADKSTSTAYMELSSLRSEDTAVYYCARNVFDGY WLVYWGQGTLVTVSS | 28 |
| B-HL13 | EVQLVESGGGVVKPGGSLRLSCAASGFTFSYSWMN WVRQAPGKGLEWMGRIFPGDGDTDYNGKFKGRVT ITADKSTSTAYMELSSLRSEDTAVYYCARNVFDGY WLVYWGQGTLVTVSS | 29 |
| B-HL14 | EVQLVESGGGLKKPGGSLRLSCAASGFTFSYSWMN WVRQAPGKGLEWMGRIFPGDGDTDYNGKFKGRVT ITADKSTSTAYMELSSLRSEDTAVYYCARNVFDGY WLVYWGQGTLVTVSS | 30 |
| B-HL15 | EVQLVESGGGLVKPGSSLRLSCAASGFTFSYSWMN WVRQAPGKGLEWMGRIFPGDGDTDYNGKFKGRVT ITADKSTSTAYMELSSLRSEDTAVYYCARNVFDGY WLVYWGQGTLVTVSS | 31 |
| B-HL16 | EVQLVESGGGLVKPGGSLRVSCAASGFTFSYSWMN WVRQAPGKGLEWMGRIFPGDGDTDYNGKFKGRVT ITADKSTSTAYMELSSLRSEDTAVYYCARNVFDGY WLVYWGQGTLVTVSS | 32 |
| B-HL17 | EVQLVESGGGLVKPGGSLRLSCAASGFTFSYSWMN WVRQAPGKGLEWMGRIFPGDGDTDYNGKFKGRVT ITADKSTSTAYMELSSLRSEDTAVYYCARNVFDGY WLVYWGQGTLVTVSS | 33 |
| VH Signal Sequence | MDWTWRILFLVAAATGAHS | 34 |
| B-KV1 | DIVMTQTPLSLPVTPGEPASISCRSSKSLLHSNGITYL YWYLQKPGQSPQLLIYQMSNLVSGVPDRFSGSGSG TDFTLKISRVEAEDVGVYYCAQNLELPYTFGGGTK VEIKRTV | 8 |
| VL Signal Sequence | MDMRVPAQLLGLLLLWFPGARC | 43 |

In some embodiments, the anti-CD20 antibody (*e.g.,* a type II anti-CD20 antibody) is an afucosylated glyco-engineered antibody. Such glycoengineered antibodies have an altered pattern of glycosylation in the Fc region, preferably having a reduced level of fucose residues. Preferably the amount of fucose is 60 % or less of the total amount of oligosaccharides at Asn297 (in one embodiment the amount of fucose is between 40 % and 60 %, in another embodiment the amount of fucose is 50 % or less, and in still another embodiment the amount of fucose is 30 % or less). Furthermore the oligosaccharides of the Fc region are preferably bisected. In some embodiments, the type II anti-CD20 antibody comprises an Fc region comprising a biantennary oligosaccharide that is bisected by N-acetyl glucosamine (GlcNAc). These glycoengineered humanized anti-CD20 (e.g., B-Ly1) antibodies have an increased ADCC.

The oligosaccharide component can significantly affect properties relevant to the efficacy of a therapeutic glycoprotein, including physical stability, resistance to protease attack, interactions with the immune system, pharmacokinetics, and specific biological activity. Such properties may depend not only on the presence or absence, but also on the specific structures, of oligosaccharides. Some generalizations between oligosaccharide structure and glycoprotein function can be made. For example, certain oligosaccharide structures mediate rapid clearance of the glycoprotein from the bloodstream through interactions with specific carbohydrate binding proteins, while others can be bound by antibodies and trigger undesired immune reactions. (Jenkins, N., et al., Nature Biotechnol. 14 (1996) 975-81).

Mammalian cells are the preferred hosts for production of therapeutic glycoproteins, due to their capability to glycosylate proteins in the most compatible form for human application. (Cumming, D.A., et al., Glycobiology 1 (1991) 115-30; Jenkins, N., et al., Nature Biotechnol. 14 (1996) 975-81). Bacteria very rarely glycosylate proteins, and like other types of common hosts, such as yeasts, filamentous fungi, insect and plant cells, yield glycosylation patterns associated with rapid clearance from the blood stream, undesirable immune interactions, and in some specific cases, reduced biological activity. Among mammalian cells, Chinese hamster ovary (CHO) cells have been most commonly used during the last two decades. In addition to giving suitable glycosylation patterns, these cells allow consistent generation of genetically stable, highly productive clonal cell lines. They can be cultured to high densities in simple bioreactors using serum free media, and permit the development of safe and reproducible bioprocesses. Other commonly used animal cells include baby hamster kidney (BHK) cells, NSO- and SP2/0-mouse myeloma cells. More recently, production from transgenic animals has also been tested. (Jenkins, N., et al., Nature Biotechnol. 14 (1996) 975-981).

Antibodies may contain carbohydrate structures at conserved positions in the heavy chain constant regions, with each isotype possessing a distinct array of N-linked carbohydrate structures, which variably affect protein assembly, secretion or functional activity. (Wright, A., and Morrison, S.L., Trends Biotech. 15 (1997) 26-32). The structure of the attached N-linked carbohydrate varies considerably, depending on the degree of processing, and can include high-mannose, multiply-branched as well as biantennary complex oligosaccharides. (Wright, A., and Morrison, S.L., Trends Biotech. 15 (1997) 26-32). Typically, there is heterogeneous processing of the core oligosaccharide structures attached at a particular glycosylation site such that even monoclonal antibodies exist as multiple glycoforms. Likewise, it has been shown that major differences in antibody glycosylation occur between cell lines, and even minor differences are seen for a given cell line grown under different culture conditions. (Lifely, M.R., et al., Glycobiology 5(8) (1995) 813-22).

One way to obtain large increases in potency, while maintaining a simple production process and potentially avoiding significant, undesirable side effects, is to enhance the natural, cell-mediated effector functions of monoclonal antibodies by engineering their oligosaccharide component as described in Umana, P., et al., Nature Biotechnol. 17 (1999) 176-180 and US 6,602,684. IgG1 type antibodies, the most commonly used antibodies in cancer immunotherapy, are glycoproteins that have a conserved N-linked glycosylation site at Asn297 in each CH2 domain. The two complex biantennary oligosaccharides attached to Asn297 are buried between the CH2 domains, forming extensive contacts with the polypeptide backbone, and their presence is essential for the antibody to mediate effector functions such as antibody dependent cellular cytotoxicity (ADCC) (Lifely, M.R., et al., Glycobiology 5 (1995) 813-822; Jefferis, R., et al., Immunol. Rev. 163 (1998) 59-76; Wright, A., and Morrison, S.L., Trends Biotechnol. 15 (1997) 26-32).

It was previously shown that overexpression in Chinese hamster ovary (CHO) cells of β(1,4)-N-acetylglucosaminyltransferase I11 ("GnTII17y), a glycosyltransferase catalyzing the formation of bisected oligosaccharides, significantly increases the in vitro ADCC activity of an antineuroblastoma chimeric monoclonal antibody (chCE7) produced by the engineered CHO cells. (See Umana, P., et al., Nature Biotechnol. 17 (1999) 176-180; and WO 99/154342, the entire contents of which are hereby incorporated by reference). The antibody chCE7 belongs to a large class of unconjugated monoclonal antibodies which have high tumor affinity and specificity, but have too little potency to be clinically useful when produced in standard industrial cell lines lacking the GnTIII enzyme (Umana, P., et al., Nature Biotechnol. 17 (1999) 176-180). That study was the first to show that large increases of ADCC activity could be obtained by engineering the antibody producing cells to express GnTIII, which also led to an increase in the proportion of constant region (Fc)-associated, bisected oligosaccharides, including bisected, non-fucosylated oligosaccharides, above the levels found in naturally-occurring antibodies.

In some embodiments, the anti-CD20 antibody (*e.g.,* a type II anti-CD20 antibody) comprises a human Fc region (*e.g.*, a human IgG1 Fc region). In some embodiments, the Fc region comprises an N-linked oligosaccharide that has been modified. In some embodiments, the N-linked oligosaccharides of the Fc region have reduced fucose residues as compared to an antibody with non-modified N-linked oligosaccharides. In some embodiments, the bisected oligosaccharide is a bisected complex oligosaccharide. In some embodiments, the N-linked oligosaccharides have been modified to have increased bisected, nonfucosylated oligosaccharides. In some embodiments, the bisected, nonfucosylated oligosaccharides are the hybrid type. In some embodiments, the bisected, nonfucosylated oligosaccharides are the complex type. For more detailed description, see, *e.g.,* WO 2003/011878 (Jean-Mairet et al.); US Patent No. 6,602,684 (Umana et al.); US 2005/0123546 (Umana et al.); and U.S. Patent No. 8,883,980 (Umana et al.).

In some embodiments, the type II anti-CD20 antibody is obinutuzumab.

### Antibody Preparation

An antibody according to any of the above embodiments (*e.g*., a type II anti-CD20 antibody of the present disclosure) may incorporate any of the features, singly or in combination, as described in Sections 1-7 below:

### 1. Antibody Affinity

In certain embodiments, an antibody provided herein has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

In one embodiment, Kd is measured by a radiolabeled antigen binding assay (RIA). In one embodiment, an RIA is performed with the Fab version of an antibody of interest and its antigen. For example, solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER^{®} multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [¹²⁵I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20^{®}) in PBS. When the plates have dried, 150 µl/well of scintillant (MICROSCINT-20 ^{™}; Packard) is added, and the plates are counted on a TOPCOUNT ^{™} gamma counter (Packard) for ten minutes. Concentrations of each Fab that give less than or equal to 20% of maximal binding are chosen for use in competitive binding assays.

According to another embodiment, Kd is measured using a BIACORE^{®} surface plasmon resonance assay. For example, an assay using a BIACORE^{®}-2000 or a BIACORE ^{®}-3000 (BIAcore, Inc., Piscataway, NJ) is performed at 25°C with immobilized antigen CM5 chips at ~10 response units (RU). In one embodiment, carboxymethylated dextran biosensor chips (CM5, BIACORE, Inc.) are activated with N-ethyl-N'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (~0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20^{™}) surfactant (PBST) at 25°C at a flow rate of approximately 25 µl/min. Association rates (kₒₙ) and dissociation rates (k_{off}) are calculated using a simple one-to-one Langmuir binding model (BIACORE ^{®} Evaluation Software version 3.2) by simultaneously fitting the association and dissociation sensorgrams. The equilibrium dissociation constant (Kd) is calculated as the ratio k_{off}/k_{on.} See, e.g., Chen et al., J. Mol. Biol. 293:865-881 (1999). If the on-rate exceeds 106 M-1 s-1 by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25oC of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO ^{™} spectrophotometer (ThermoSpectronic) with a stirred cuvette.

### 2. Antibody Fragments

In certain embodiments, an antibody provided herein is an antibody fragment. Antibody fragments include, but are not limited to, Fab, Fab', Fab'-SH, F(ab')₂, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')₂ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

### 3. Chimeric and Humanized Antibodies

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 4. Human Antibodies

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE^{™} technology; U.S. Patent No. 5,770,429 describing HUMAB^{®} technology; U.S. Patent No. 7,041,870 describing K-M MOUSE^{®} technology, and U.S. Patent Application Publication No. US 2007/0061900, describing VELOCIMOUSE^{®} technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro*, as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Multispecific Antibodies

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for CD20 and the other is for any other antigen. In certain embodiments, bispecific antibodies may bind to two different epitopes of CD20. Bispecific antibodies may also be used to localize cytotoxic agents to cells which express CD20. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising an antigen binding site that binds to CD20 as well as another, different antigen (see, US 2008/0069820, for example).

### 7. Antibody Variants

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### a) Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table A under the heading of "preferred substitutions." More substantial changes are provided in Table A under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**TABLE A**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (*e.g*. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

### b) Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams *et al*., especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### c) Fc region variants

In certain embodiments, one or more amino acid modifications may be introduced into the Fc region of an antibody provided herein, thereby generating an Fc region variant. The Fc region variant may comprise a human Fc region sequence (*e.g.,* a human IgG1, IgG2, IgG3 or IgG4 Fc region) comprising an amino acid modification (*e.g.* a substitution) at one or more amino acid positions.

In certain embodiments, the invention contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express Fc(RIII only, whereas monocytes express Fc(RI, Fc(RII and Fc(RIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI^{™} non-radioactive cytotoxicity assay for flow cytometry (CellTechnology, Inc. Mountain View, CA; and CytoTox 96^{®} non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo*, *e.g*., in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

In certain embodiments, the Fc variants described herein further comprise one or more amino acid modifications for attenuating effector function (such as CDC and/or ADCC). In exemplary embodiments, the modification to attenuate effector function is a modification that does not alter the glycosylation pattern of the Fc region. In certain embodiments, the modification to attenuate effector function reduces or eliminates binding to human effector cells, binding to one or more Fc receptors, and/or binding to cells expressing an Fc receptor. In an exemplary embodiment, the Fc variants described herein comprise the following modifications: L234A, L235A and P329G in the Fc region of human IgG1, that result in attenuated effector function. Substitutions L234A, L235A, and P329G (the L234A/L235A/P329G triple variant is referred to as LALAPG) have previously been shown to reduce binding to Fc receptors and complement (see e.g., US Publication No. 2012/0251531).

In various embodiments, Fc variants having reduced effector function refer to Fc variants that reduce effector function (e.g., CDC, ADCC, and/or binding to FcR, etc. activities) by at least 10%, 20%, 30%, 40%, 50%, 60%, 70%, 80%, 90%, 95%, 97%, 98%, 99% or more as compared to the effector function achieved by a wild-type Fc region (e.g., an Fc region not having a mutation to reduce effector function, although it may have other mutations). In certain embodiments, Fc variants having reduced effector function refer to Fc variants that eliminate all detectable effector function as compared to a wild-type Fc region. Assays for measuring effector function are known in the art and described below.

*In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity). The primary cells for mediating ADCC, NK cells, express FcyRIII only, whereas monocytes express FcyRI, FcyRII and FcyRIII. FcR expression on hematopoietic cells is summarized in Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI^{™} non-radioactive cytotoxicity assay for flow cytometry (CellTechnology, Inc. Mountain View, CA; and CytoTox 96^{®} non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)).

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (*i.e.*, either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

### d) Cysteine engineered antibody variants

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

### e) Antibody Derivatives

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### A. Recombinant Methods and Compositions

Antibodies may be produced using recombinant methods and compositions, e.g., as described in U.S. Patent No. 4,816,567. In one embodiment, isolated nucleic acid encoding an anti-CD20 antibody described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp20 cell). In one embodiment, a method of making an anti-CD20 antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-CD20 antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli*.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES^{™} technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

### B. Assays

Anti-CD20 antibodies provided herein may be identified, screened for, or characterized for their physical/chemical properties and/or biological activities by various assays known in the art.

### 1. Binding assays and other assays

In one aspect, an antibody of the invention is tested for its antigen binding activity, e.g., by known methods such as ELISA, Western blot, etc. CD20 binding may be determined using methods known in the art and exemplary methods are disclosed herein. In one embodiment, binding is measured using radioimmunoassay. An exemplary radioimmunoassay is provided below. CD20 antibody is iodinated, and competition reaction mixtures are prepared containing a fixed concentration of iodinated antibody and decreasing concentrations of serially diluted, unlabeled CD20 antibody. Cells expressing CD20 (e.g., BT474 cells stably transfected with human CD20) are added to the reaction mixture. Following an incubation, cells are washed to separate the free iodinated CD20 antibody from the CD20 antibody bound to the cells. Level of bound iodinated CD20 antibody is determined, e.g., by counting radioactivity associated with cells, and binding affinity determined using standard methods. In another embodiment, ability of CD20 antibody to bind to surface-expressed CD20 (e.g., on B cell subsets) is assessed using flow cytometry. Peripheral white blood cells are obtained (e.g., from human, cynomolgus monkey, rat or mouse) and cells are blocked with serum. Labeled CD20 antibody is added in serial dilutions, and T cells are also stained to identify T cell subsets (using methods known in the art). Following incubation of the samples and washing, the cells are sorted using flow cytometer, and data analyzed using methods well known in the art. In another embodiment, CD20 binding may be analyzed using surface plasmon resonance. An exemplary surface plasmon resonance method is exemplified in the Examples.

In another aspect, competition assays may be used to identify an antibody that competes with any of the anti-CD20 antibodies disclosed herein for binding to CD20. In certain embodiments, such a competing antibody binds to the same epitope (e.g., a linear or a conformational epitope) that is bound by any of the anti-CD20 antibodies disclosed herein. Detailed exemplary methods for mapping an epitope to which an antibody binds are provided in Morris (1996) "Epitope Mapping Protocols," in Methods in Molecular Biology vol. 66 (Humana Press, Totowa, NJ).

In an exemplary competition assay, immobilized CD20 is incubated in a solution comprising a first labeled antibody that binds to CD20 (e.g., rituximab, a GA101 antibody, etc.) and a second unlabeled antibody that is being tested for its ability to compete with the first antibody for binding to CD20. The second antibody may be present in a hybridoma supernatant. As a control, immobilized CD20 is incubated in a solution comprising the first labeled antibody but not the second unlabeled antibody. After incubation under conditions permissive for binding of the first antibody to CD20, excess unbound antibody is removed, and the amount of label associated with immobilized CD20 is measured. If the amount of label associated with immobilized CD20 is substantially reduced in the test sample relative to the control sample, then that indicates that the second antibody is competing with the first antibody for binding to CD20. See Harlow and Lane (1988) Antibodies: A Laboratory Manual ch.14 (Cold Spring Harbor Laboratory, Cold Spring Harbor, NY).

### 2. Activity assays

Anti-CD20 antibodies of the present disclosure (*e.g.,* a type II antibody) may be identified and/or characterized by one or more activity assays known in the art. For example, a complement-dependent cytotoxicity (CDC) and/or antibody-dependent cellular cytotoxicity (ADCC) may be used, as described herein.

It is understood that any of the above assays may be carried out using an immunoconjugate of the invention in place of or in addition to an anti-CD20 antibody.

It is understood that any of the above assays may be carried out using anti-CD20 antibody and an additional therapeutic agent.

### Methods of Administering a type II anti-CD20 antibody

Provided herein are methods for treating childhood-onset idiopathic nephrotic syndrome (INS) in an individual, wherein the methods comprise administering to the individual a first antibody exposure to a type II anti-CD20 antibody and a second antibody exposure to the type II anti-CD20 antibody. Also provided herein are methods for depleting circulating peripheral B cells in an individual, wherein the methods comprise administering to the individual a first antibody exposure to a type II anti-CD20 antibody, and a second antibody exposure to the type II anti-CD20 antibody, and wherein after administration of the type II anti-CD20 antibody, B cells are depleted to a level such that circulating peripheral B cells are present in peripheral blood from the individual at about 5 cells/µL or fewer. Also provided herein are methods for depleting circulating peripheral B cells in an individual, wherein the methods comprise administering to the individual a first antibody exposure to a type II anti-CD20 antibody and a second antibody exposure to the type II anti-CD20 antibody, and wherein after administration of the type II anti-CD20 antibody, B cells are depleted to a level such that circulating peripheral B cells are present in peripheral blood from the individual at about 5 cells/µL or fewer which is sustained for at least 52 weeks after the first dose of the first antibody exposure. In some embodiments of the methods herein, the individual or patient is a human. In some embodiments, the individual or patient is a human that is greater than or equal to 2 years of age and less than or equal to 25 years of age. In some embodiments, the individual or patient is a human that is greater than 2 years of age and less than 25 years of age. In some embodiments, *e*.*g*., using weight-based dosing of the type II anti-CD20 antibody, the individual weighs less than 45kg. In some embodiments, *e*.*g*., using fixed dosing of the type II anti-CD20 antibody, the individual weighs greater than or equal to 45kg.

In some embodiments, the individual or patient has been diagnosed with INS (*e.g.,* FRNS or SDNS) before the age of 18 years. Guidelines for diagnosing childhood-onset INS (*e.g.,* FRNS or SDNS) are known in the art and include, without limitation, those described in Kidney Disease: Improving Global Outcomes Glomerular Diseases Work Group. KDIGO 2021 Clinical Practice Guideline for the Management of Glomerular Diseases (Kidney Int. 2021; 100:S1-276).

In some embodiments, the individual or patient has childhood-onset frequently relapsing nephrotic syndrome (FRNS). In some embodiments, the individual or patient has had ≥2 relapses per 6 months within 6 months of disease onset or ≥4 relapses per 12 months in any subsequent 12 month period.

In some embodiments, the individual or patient has childhood-onset steroid-dependent nephrotic syndrome (SDNS). In some embodiments, the individual or patient has had two consecutive relapses during therapy with prednisone or prednisolone (either at full dose or during tapering) or within 15 days of prednisone or prednisolone discontinuation.

In some embodiments, the individual or patient is in complete remission, *e*.*g*., prior to treatment using the methods of the present disclosure. In some embodiments, complete remission is defined as the absence of edema, UPCR ≤0.2g/g, and 3 consecutive daily urine dipstick readings of trace or negative for protein.

In some embodiments, the individual or patient has had at least 1 relapse within 6 months, *e*.*g*., prior to treatment according to the methods of the present disclosure.

In some embodiments, the individual or patient has received cyclophosphamide within 6 months, *e*.*g*., prior to treatment according to the methods of the present disclosure, and has experienced at least 1 relapse subsequent to cyclophosphamide discontinuation.

In some embodiments, the individual or patient has an estimated glomerular filtration rate (eGFR) within normal range for their age.

In some embodiments, the methods of the present disclosure include administering to the individual a first antibody exposure to a type II anti-CD20 antibody of the present disclosure and a second antibody exposure to the type II anti-CD20 antibody of the present disclosure. In some embodiments, the second antibody exposure is not provided until from about 18 weeks to about 26 weeks after the first antibody exposure. In some embodiments, the second antibody exposure is not provided until about 18 weeks after the first antibody exposure, about 19 weeks after the first antibody exposure, about 20 weeks after the first antibody exposure, about 21 weeks after the first antibody exposure, about 22 weeks after the first antibody exposure, about 23 weeks after the first antibody exposure, about 24 weeks after the first antibody exposure, about 25 weeks after the first antibody exposure, or about 26 weeks after the first antibody exposure. In some embodiments, the second antibody exposure is not provided until less than about any of the following weeks after the first antibody exposure: 26, 25, 24, 23, 22, 21, 20, or 19. In some embodiments, the second antibody exposure is not provided until greater than about any of the following weeks after the first antibody exposure: 18, 19, 20, 21, 22, 23, 24, or 25. That is, the second antibody exposure is not provided until any of a range of weeks having an upper limit of 26, 25, 24, 23, 22, 21, 20, or 19 and an independently selected lower limit of 18, 19, 20, 21, 22, 23, 24, or 25, wherein the lower limit is less than the upper limit.

The dosing regimens described herein use a consistent system for tracking time between doses whereby the first dose is administered to the patient on Day 1 or week 0. As described herein, an antibody exposure of the present disclosure may include one or two doses. In cases where the antibody exposures contain one dose, references to a second antibody exposure not provided until a period of time has elapsed after a first antibody exposure (as described herein) refer to the amount of time elapsed between the dose of the first antibody exposure (*e.g.,* Day 1 or week 0) and the dose of the second antibody exposure. If the first antibody exposure includes two doses, the first dose of the first antibody exposure is provided on Day 1 or week 0. In cases where the antibody exposures contain two doses, references to a second antibody exposure not provided until a period of time has elapsed after a first antibody exposure (as described herein) refer to the amount of time elapsed between the first of the two doses of the first antibody exposure (*e.g.,* Day 1 or week 0) and the first dose of the two doses of the second antibody exposure. For example, if a method of the present disclosure includes a first antibody exposure with two doses and a second antibody exposure with two doses, and the second antibody exposure is not provided until about 22 weeks after the first antibody exposure, then the interval between the first dose of the first antibody exposure and the first dose of the second antibody exposure is about 22 weeks.

In some embodiments, a first antibody exposure of the present disclosure includes one or two doses of a type II anti-CD20 antibody of the present disclosure. In some embodiments, the first antibody exposure contains a total exposure of between about 1800mg and about 2200mg of the type II anti-CD20 antibody. In some embodiments, the first antibody exposure contains a total exposure of about 1800mg, about 1900mg, about 2000mg, about 2100mg, or about 2200mg of the type II anti-CD20 antibody. In some embodiments, the individual weighs greater than or equal to 45kg.

In some embodiments, the first antibody exposure contains a total exposure of between about 36mg/kg and about 44mg/kg of the type II anti-CD20 antibody. In some embodiments, the first antibody exposure contains a total exposure of about 36mg/kg, about 38mg/kg, about 40mg/kg, about 42mg/kg, or about 44mg/kg of the type II anti-CD20 antibody. In some embodiments, the individual weighs less than 45kg.

In some embodiments, the first antibody exposure includes two doses. In some embodiments, the first antibody exposure includes a first dose of between about 900mg and about 1100mg of the type II anti-CD20 antibody and a second dose of between about 900mg and about 1100mg of the type II anti-CD20 antibody. In some embodiments, the first dose of the first antibody exposure contains about 1000mg of the type II anti-CD20 antibody. In some embodiments, the second dose of the first antibody exposure contains about 1000mg of the type II anti-CD20 antibody. In some embodiments, the individual weighs greater than or equal to 45kg.

In some embodiments, the first antibody exposure includes two doses. In some embodiments, the first antibody exposure includes a first dose of between about 18mg/kg and about 22mg/kg of the type II anti-CD20 antibody and a second dose of between about 18mg/kg and about 22mg/kg of the type II anti-CD20 antibody. In some embodiments, the first dose of the first antibody exposure contains about 20mg/kg of the type II anti-CD20 antibody. In some embodiments, the second dose of the first antibody exposure contains about 20mg/kg of the type II anti-CD20 antibody. In some embodiments, the individual weighs less than 45kg.

In some embodiments, the second dose of the first antibody exposure is not provided until about 1.5 weeks to about 2.5 weeks after the first dose of the first antibody exposure. In some embodiments, the second dose of the first antibody exposure is not provided until about 2 weeks after the first dose of the first antibody exposure.

In some embodiments, a second antibody exposure of the present disclosure includes one or two doses of a type II anti-CD20 antibody of the present disclosure. In some embodiments, the second antibody exposure contains a total exposure of between about 1800mg and about 2200mg of the type II anti-CD20 antibody. In some embodiments, the second antibody exposure contains a total exposure of about 1800mg, about 1900mg, about 2000mg, about 2100mg, or about 2200mg of the type II anti-CD20 antibody. In some embodiments, the individual weighs greater than or equal to 45kg.

In some embodiments, the second antibody exposure contains a total exposure of between about 36mg/kg and about 44mg/kg of the type II anti-CD20 antibody. In some embodiments, the second antibody exposure contains a total exposure of about 36mg/kg, about 38mg/kg, about 40mg/kg, about 42mg/kg, or about 44mg/kg of the type II anti-CD20 antibody. In some embodiments, the individual weighs less than 45kg.

In some embodiments, the second antibody exposure includes two doses. In some embodiments, the second antibody exposure includes a first dose of between about 900mg and about 1100mg of the type II anti-CD20 antibody and a second dose of between about 900mg and about 1100mg of the type II anti-CD20 antibody. In some embodiments, the first dose of the second antibody exposure contains about 1000mg of the type II anti-CD20 antibody. In some embodiments, the second dose of the second antibody exposure contains about 1000mg of the type II anti-CD20 antibody. In some embodiments, the individual weighs greater than or equal to 45kg.

In some embodiments, the second antibody exposure includes two doses. In some embodiments, the second antibody exposure includes a first dose of between about 18mg/kg and about 22mg/kg of the type II anti-CD20 antibody and a second dose of between about 18mg/kg and about 22mg/kg of the type II anti-CD20 antibody. In some embodiments, the first dose of the second antibody exposure contains about 20mg/kg of the type II anti-CD20 antibody. In some embodiments, the second dose of the second antibody exposure contains about 20mg/kg of the type II anti-CD20 antibody. In some embodiments, the individual weighs less than 45kg.

In some embodiments, the second dose of the second antibody exposure is not provided until about 1.5 weeks to about 2.5 weeks after the first dose of the second antibody exposure. In some embodiments, the second dose of the second antibody exposure is not provided until about 2 weeks after the first dose of the second antibody exposure.

In some embodiments, a type II anti-CD20 antibody of the present disclosure is administered intravenously (*e.g.,* by IV infusion).

In some embodiments, the methods of the present disclosure further include administering an effective amount of a glucocorticoid or corticosteroid (*e.g.,* in conjunction with a type II anti-CD20 antibody as described herein). A variety of naturally occurring and synthetic glucocorticoids/corticosteroids are known in the art, including without limitation beclometasone, triamcinolone, dexamethasone, betamethasone, prednisone, methylprednisolone, prednisolone, cortisone, and cortisol. In some embodiments, the glucocorticoids/corticosteroid includes methylprednisolone. In some embodiments, the glucocorticoids/corticosteroid includes prednisone. Effective amounts of the glucocorticoids/corticosteroids of the present disclosure are known in the art and readily ascertainable by standard assays. For example, methylprednisolone may be administered at 750-1000mg doses once daily by IV. As another example, prednisone may be administered orally at 0.5mg/kg and optionally tapered to 7.5mg/day. In some embodiments, methylprednisolone may be administered prior to each anti-CD20 antibody infusion. In some embodiments, methylprednisolone may be administered intravenously at 80mg (*e.g.*, if the individual weighs greater than or equal to 45kg) or 1.5mg/kg (*e.g.*, if the individual weighs less than 45kg). In some embodiments, oral prednisone or an equivalent may be administered at a dose of 0.5-1mg/kg/day (maximum 60mg/day). In some embodiments, oral prednisone or an equivalent may be administered at a dose of 0.5-1mg/kg/day (maximum 60mg/day) and tapered to a goal of 5mg/day. In some embodiments, oral prednisone or an equivalent may be administered at a dose of 0.5-2mg/kg/day (maximum 60mg/day). In some embodiments, oral prednisone or an equivalent may be administered at a dose of 0.5-2mg/kg/day (maximum 60mg/day) and tapered to a goal of 5mg/day.

In some embodiments, a glucocorticoid may be administered before, during, or after administration of a type II anti-CD20 antibody of the present disclosure. In some embodiments, a glucocorticoid may be administered prior to administration of a type II anti-CD20 antibody of the present disclosure, *e.g.,* 30-60 minutes before the type II anti-CD20 antibody. In some embodiments, 80mg methylprednisolone may be administered by IV 30-60 minutes before administration of a type II anti-CD20 antibody of the present disclosure. In some embodiments, prednisone (*e.g.,* orally administered) and/or methyl prednisolone (*e.g.,* IV administered) may be administered with treatment, followed by a maintenance treatment (*e.g.,* mycophenolate mofetil or cyclophosphamide).

In some embodiments, the methods of the present disclosure further include administering an effective amount of an antihistamine (*e.g.,* in conjunction with a type II anti-CD20 antibody as described herein). Antihistamines known in the art and currently in clinical use include histamine H₁-receptor and histamine H₂-receptor antagonists or inverse agonists. In some embodiments, the antihistamine includes diphenhydramine. Effective amounts of the antihistamines of the present disclosure are known in the art and readily ascertainable by standard assays. For example, diphenhydramine may be administered in at 0.5-1mg/kg oral doses (rounded to nearest available pill formulation), up to a maximum dose of 50mg.

In some embodiments, an antihistamine may be administered before, during, or after administration of a type II anti-CD20 antibody of the present disclosure, *e.g.,* as a prophylactic treatment. In some embodiments, an antihistamine may be administered prior to administration of a type II anti-CD20 antibody of the present disclosure, *e.g.,* 30-60 minutes before the type II anti-CD20 antibody. In some embodiments, 0.5-1mg/kg or up to 50mg diphenhydramine may be administered orally 30-60 minutes before administration of a type II anti-CD20 antibody of the present disclosure.

In some embodiments, the methods of the present disclosure further include administering an effective amount of acetaminophen. For example, acetaminophen may be administered at 15mg/kg oral doses, up to a maximum dose of 1000mg.

In some embodiments, acetaminophen may be administered before, during, or after administration of a type II anti-CD20 antibody of the present disclosure, *e.g.,* as a prophylactic treatment. In some embodiments, acetaminophen may be administered prior to administration of a type II anti-CD20 antibody of the present disclosure, *e.g.,* 30-60 minutes before the type II anti-CD20 antibody. In some embodiments, 15mg/kg (rounded to nearest available pill formulation) or up to 1000mg acetaminophen may be administered orally 30-60 minutes before administration of a type II anti-CD20 antibody of the present disclosure.

In some embodiments, the methods of the present disclosure further include administering a standard of care treatment (*e*.*g*., in conjunction with a type II anti-CD20 antibody as described herein). In some embodiments, a standard of care treatment may be administered before, during, or after administration of a type II anti-CD20 antibody of the present disclosure, *e.g.,* for treating or preventing one or more symptoms of INS.

In some embodiments, the methods of the present disclosure result in a complete remission in the individual. In some embodiments, the individual is in complete remission at 1 year after commencement of treatment, *e*.*g*., at Week 52 as described herein. In some embodiments, complete remission refers to the state of having a first morning void UPCR ≤0.2g/g without occurrence of an intercurrent event (occurring after Week 8), *e.g.,* as described herein, after completion of steroid taper. In some embodiments, the complete remission is sustained, *e.g.,* at 1 year after commencement of treatment, *e.g.,* at Week 52 as described herein. In some embodiments, a sustained complete remission includes a first morning void UPCR ≤0.2g/g without occurrence of relapse or any of certain intercurrent events: (1) relapse *(e.g.,* occurring after Week 8) as defined by any of the following events requiring systemic corticosteroid or other immunosuppressive treatment: (a) first morning void UPCR≥2 g/g or (b) dipstick UA ≥3+ for 3 consecutive days and the most recent urine sample of the 3-day period determined to have UPCR > 0.2 g/g or (c) dipstick UA protein ≥3+ on any single day with edema and urine sample determined to have UPCR > 0.2g/g; (2) any systemic corticosteroid usage for > 14 days in a 30-day period (*e*.*g*., occurring after Week 8); (3) initiation of any rescue therapy for INS other than systemic corticosteroids (*e*.*g*., occurring at any time); (4) treatment discontinuation due to lack of efficacy (*e.g.,* occurring at any time); or (5) death (*e.g.,* occurring at any time). In some embodiments, the complete remission is sustained in the individual from Week 8 to Week 52 of treatment without the individual experiencing (1) relapse as defined by any of the following events requiring systemic corticosteroid or other immunosuppressive treatment: (a) first morning void UPCR≥2 g/g or (b) dipstick UA ≥3+ for 3 consecutive days and the most recent urine sample of the 3-day period determined to have UPCR > 0.2 g/g or (c) dipstick UA protein ≥3+ on any single day with edema and urine sample determined to have UPCR > 0.2g/g; or (2) any systemic corticosteroid usage for >14 days in a 30-day period. In some embodiments, the complete remission is sustained in the individual until Week 52 of treatment without initiation of any rescue therapy for INS other than systemic corticosteroids.

In some embodiments, the methods of the present disclosure result in a depletion of circulating peripheral B cells in an individual. In some embodiments, the circulating peripheral B cells are CD19+ B cells. In some embodiments, the circulating peripheral B cells are Naïve B cells. In some embodiments, the circulating peripheral B cells are Memory B cells. In some embodiments, the circulating peripheral B cells are Plasmablasts or Plasma cells. In some embodiments, after administration of a type II anti-CD20 antibody of the present disclosure (*e.g.,* according to any of the methods described herein), circulating peripheral B cells are present in peripheral blood at about about 7 cells/µL or fewer, about 6 cells/µL or fewer, about 5 cells/µL or fewer, about 4 cells/µL or fewer, about 3 cells/µL or fewer, about 2 cells/µL or fewer, about 1 cell/µL or fewer, or about 0.5 cells/ µL or fewer. In some embodiments, the level of circulating peripheral B cells are measured using highly sensitive flow cytometry (HSFC) described herein. In some embodiments, B cells are depleted to a level that is below the detectable limit using HSFC. In some embodiments, the HSFC has a lower limit of quantitation (LLOQ) for B cells of about 1.0 cells/µL or fewer, about 0.8 cells/µL or fewer, about 0.6 cells/µL or fewer, about 0.5 cells/µL or fewer, or 0.441 cells/µL or fewer. In some embodiments, circulating peripheral B cells in the individual are depleted by at least about 50%, at least about 60%, at least about 70%, at least about 80%, at least about 90%, or about 100%. In some embodiments, depletion of circulating peripheral B cells is sustained for at least 52 weeks after the first dose of the first antibody exposure. In some embodiments, depletion of circulating peripheral B cells is sustained for at least 51 weeks, at least 50 weeks, at least 49 weeks, at least 48 weeks, at least 47 weeks, at least 46 weeks, at least 45 weeks, at least 44 weeks, at least 43 weeks, at least 42 weeks, at least 41 weeks, at least 40 weeks, at least 39 weeks, at least 38 weeks, at least 37 weeks, at least 36 weeks, at least 35 weeks, at least 34 weeks, at least 33 weeks, at least 32 weeks, at least 31 weeks, at least 30 weeks, at least 29 weeks, at least 28 weeks, at least 27 weeks, at least 26 weeks, at least 25 weeks, or at least 24 weeks after the first dose of the first antibody exposure. In some embodiments, depletion of circulating peripheral B cells refers to a measurement of circulating peripheral B cells taken after a first antibody exposure (*e.g.,* including 1 or 2 doses of an anti-CD20 antibody as described herein), after a second antibody exposure (*e.g.,* including 1 or 2 doses of an anti-CD20 antibody as described herein), 3 months after treatment (*e.g.,* after receiving a first and/or second antibody exposure as described herein), 6 months after treatment (*e.g.,* after receiving a first and/or second antibody exposure as described herein), 9 months after treatment (*e.g.,* after receiving a first and/or second antibody exposure as described herein), or 12 months after treatment (*e.g.,* after receiving a first and/or second antibody exposure as described herein), *e.g.,* as compared to a corresponding measurement in the same individual before treatment, or as compared to a corresponding measurement in a control individual (*e.g.,* an individual that has not received treatment).

Methods for assaying depletion of circulating peripheral B cells in an individual are known in the art, *e.g.,* flow cytometry using one or more antibodies that recognize a B cell marker. In some embodiments, highly sensitive flow cytometry (HSFC) may be used to assay depletion of circulating peripheral B cells (see, *e.g.,* Vital, E.M. et al. (2011) Arthritis Rheum. 63:3038-3047 and Example 1). In some embodiments, the B cells are CD19+ B cells. In some embodiments, the B cells are naïve B cells (*e.g.,* CD19+ CD27- B cells), memory B cells (*e.g.,* CD19+ CD27+ B cells), or plasmablasts (*e.g.,* CD19+ CD27+ CD38++ B cells). In some embodiments, the B cells are CD19+CD3-CD14- cells and/or CD19+CD33-CD56- cells. In some embodiments, the B cells are CD19+CD3-CD14-CD33-CD56- cells. In some embodiments, the B cells comprise CD19+CD20+ B cells, CD19+CD20- B cells, and CD19+CD22+ B cells. In some embodiments, the B cells are circulating peripheral B cells, *e.g.*, from a peripheral blood sample.

In some embodiments, level of circulating peripheral B cells present in a peripheral blood sample is measured (*e.g.*, by HSFC) as follows. Lymphocytes are identified in a sample by flow cytometry (*e.g.*, by plotting CD45 vs. side scatter and gating CD45+ cells). In some embodiments, doublets are excluded from analysis prior to this step (*e.g.*, by gating single cells and excluding forward scatter and/or side scatter doublets). CD19+ B cells are then identified by excluding T cells, NK cells, and monocytes. For example, CD19+CD3-CD14- cells can be identified from a parent CD45+ lymphocyte gate (*e.g.*, by plotting CD19 vs. CD3/CD14 and gating CD19+CD3-CD14- cells), and CD19+CD33-CD56- B cells can be identified from a parent CD19+CD3-CD14- cells (*e.g.*, by plotting CD19 vs. CD33/CD56 and gating CD19+CD33-CD56- cells). B cell counts can then be determined, *e.g.*, by dividing the number of CD19+ B cells detected (*e.g.,* CD19+CD3-CD14-CD33-CD56- cells) by the sample volume. In some embodiments, a number of beads or other QC control is also quantified, and B cell counts can then be determined, *e.g.*, by calculating (CD19+ events x bead count)/(bead count x sample volume).

In some embodiments, after administration of a type II anti-CD20 antibody of the present disclosure (*e.g.,* according to any of the methods described herein), circulating peripheral B cells are present in peripheral blood at about 7 cells/µL or fewer, about 6 cells/µL or fewer, about 5 cells/µL or fewer, about 4 cells/µL or fewer, about 3 cells/µL or fewer, about 2 cells/µL or fewer, about 1 cell/µL or fewer, or about 0.5 cells/ µL or fewer, *e.g*., 5 cells/µL or fewer. In some embodiments, B cells are depleted to a level that is below the detectable limit using HSFC. In some embodiments, the HSFC has a lower limit of quantitation (LLOQ) for B cells of about 1.0 cells/µL or fewer, about 0.8 cells/µL or fewer, about 0.6 cells/µL or fewer, about 0.5 cells/µL or fewer, or 0.441 cells/µL or fewer.

### IV. Articles of Manufacture or Kits

In another aspect, an article of manufacture or kit containing a type II anti-CD20 antibody of the present disclosure useful in any of the methods described herein (e.g., for the treatment, prevention and/or diagnosis of the disorders described herein) is provided. The article of manufacture or kit comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition or for depleting circulating peripheral B cells and may have a sterile access port (for example the container may be an intravenous solution bag or a vial having a stopper pierceable by a hypodermic injection needle). At least one active agent in the composition is an antibody described herein (*e.g.,* a type II anti-CD20 antibody of the present disclosure). The label or package insert indicates that the composition is used for treating the condition of choice or for depleting circulating peripheral B cells according to any of the methods described herein. Alternatively, or additionally, the article of manufacture or kit may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

In some embodiments, provided herein is an article of manufacture or a kit comprising a container comprising a type II anti-CD20 antibody of the present disclosure and an optional pharmaceutically acceptable carrier, and, optionally, a package insert comprising instructions for treating childhood-onset INS in an individual or reducing risk and/or frequency of relapse of childhood-onset INS in an individual*, e.g.,* wherein the instructions indicate that a first antibody exposure to a type II anti-CD20 antibody and a second antibody exposure to the type II anti-CD20 antibody are administered to the individual, the second antibody exposure not being provided until from about 18 weeks to about 26 weeks after the first antibody exposure; wherein the first antibody exposure comprises one or two doses of the type II anti-CD20 antibody, the first antibody exposure comprising a total exposure of between about 1800mg and about 2200mg of the type II anti-CD20 antibody; wherein the second antibody exposure comprises one or two doses of the type II anti-CD20 antibody, the second antibody exposure comprising a total exposure of between about 1800mg and about 2200mg of the type II anti-CD20 antibody. In some embodiments, the instructions indicate that the individual is greater than or equal to 2 years of age and less than or equal to 25 years of age. In some embodiments, the instruction indicate that the individual weighs greater than or equal to 45kg. In some embodiments, the antibody is obinutuzumab.

In some embodiments, provided herein is an article of manufacture or a kit comprising a container comprising a type II anti-CD20 antibody of the present disclosure and an optional pharmaceutically acceptable carrier, and, optionally, a package insert comprising instructions for treating childhood-onset INS in an individual or reducing risk and/or frequency of relapse of childhood-onset INS in an individual*, e.g.,* wherein the instructions indicate that a first antibody exposure to a type II anti-CD20 antibody and second antibody exposure to the type II anti-CD20 antibody are administered to the individual, the second antibody exposure not being provided until from about 18 weeks to about 26 weeks after the first antibody exposure; wherein the first antibody exposure comprises one or two doses of the type II anti-CD20 antibody, the first antibody exposure containing a total exposure of between about 36mg/kg and about 44mg/kg of the type II anti-CD20 antibody; wherein the second antibody exposure comprises one or two doses of the type II anti-CD20 antibody, the second antibody exposure containing a total exposure of between about 36mg/kg and about 44mg/kg of the type II anti-CD20 antibody. In some embodiments, the instructions indicate that the individual is greater than or equal to 2 years of age and less than or equal to 25 years of age. In some embodiments, the instructions indicate that the individual weighs less than 45kg. In some embodiments, the antibody is obinutuzumab.

The article of manufacture or kit may still further comprise a second or third container comprising a second medicament, wherein the anti-CD20 antibody (*e.g*., a type II anti-CD20 antibody of the present disclosure) is a first medicament, where the article further comprises instructions on the package insert for treating the subject with the second medicament. The article of manufacture in these embodiments may further comprise a package insert indicating that the compositions can be used to treat a particular condition.

The specification is considered to be sufficient to enable one skilled in the art to practice the invention. Various modifications of the invention in addition to those shown and described herein will become apparent to those skilled in the art from the foregoing description and fall within the scope of the appended claims. All publications, patents, and patent applications cited herein are hereby incorporated by reference in their entirety for all purposes.

### EXAMPLES

The invention will be more fully understood by reference to the following examples. They should not, however, be construed as limiting the scope of the invention. It is understood that the examples and embodiments described herein are for illustrative purposes only and that various modifications or changes in light thereof will be suggested to persons skilled in the art and are to be included within the spirit and purview of this application and scope of the appended claims.

### Example 1: A Phase III, multicenter, randomized open label study to evaluate the efficacy and safety of obinutuzumab versus MMF in patients with childhood onset idiopathic nephrotic syndrome

A Phase III, randomized, open label, multicenter, active comparator study to evaluate the efficacy, safety, and pharmacokinetics (PK)/pharmacodynamics (PD) of obinutuzumab versus MMF in maintaining remission in participants with childhood-onset frequently relapsing nephrotic syndrome (FRNS) or steroid-dependent nephrotic syndrome (SDNS) who have achieved complete remission upon study entry and are considered to be at high risk of relapse is presented below.

Patients with childhood onset FRNS/SDNS are at risk of short-term and long-term steroid toxicity, severe infections, edema, thromboembolic events, and acute kidney injury during relapses. The reduction of these risks is dependent on the identification of effective therapy and the maintenance of clinical remission via sustained reduction in proteinuria. Current standard of care remains limited to a combination of systemic corticosteroids and immunosuppressive therapies, but most available regimens confer a complete renal response and remission in fewer than half of treated patients.

### Objectives and endpoints

This study evaluates the efficacy, safety, pharmacokinetics, and pharmacodynamics of obinutuzumab compared with MMF in participants aged between ≥ 2 and ≤ 25 years old with childhood onset FRNS or SDNS. Specific primary and secondary objectives and corresponding endpoints for the study are outlined below. In this protocol, "study treatment" refers to the treatments assigned to participants as part of this study (*i.e*., obinutuzumab or MMF).

The primary objective of the study is to evaluate the efficacy of obinutuzumab compared with MMF in participants aged between ≥ 2 and ≤ 25 years old with childhood onset FRNS or SDNS. The primary endpoint is the proportion of participants achieving sustained complete remission at 1 year, defined as first morning void urinary protein to creatinine ratio (UPCR) ≤ 0.2 g/g at Week 52 without occurrence of a relapse or any of the following intercurrent events. Intercurrent events occurring after Week 8 include : (1) relapse as defined by any of the following events: (a) first-morning void UPCR ≥ 2 g/g, (b) Dipstick urinalysis (UA) ≥ 3+ for 3 consecutive days (home-monitoring) and the most recent urine sample of the 3-day period determined to have UPCR > 0.2 g/g as measured by the central laboratory, or (c) Dipstick UA protein ≥ 3+ on any single day with edema and urine sample determined to have UPCR > 0.2 g/g as measured by the central laboratory; and (2) any systemic corticosteroids usage for > 14 days in a 30 day period. Intercurrent events occurring after randomization include: (1) initiation of any rescue therapy for idiopathic nephrotic syndrome (INS) as determined by the investigators best medical judgment, other than systemic corticosteroids; (2) treatment discontinuation due to lack of efficacy; and/or (3) death.

A secondary objective is to evaluate the efficacy of obinutuzumab compared with MMF. Corresponding secondary endpoints are: (1) overall relapse free survival (RFS); (2) probability of RFS at Week 52; (3) cumulative corticosteroid dose; (4) number of relapses on randomized study treatment; (5) proportion of participants experiencing edema associated relapse during the 52-week treatment period; and (6) proportion of patients with sustained complete remission at Week 76.

Another secondary objective is to evaluate changes in fatigue of participants treated with obinutuzumab compared with MMF. The corresponding secondary endpoint is the mean change in "General Fatigue" domain of Pediatric Quality of Life Inventory (PedsQL) Multidimensional Fatigue scale total score from baseline to Week 52.

The third secondary objective is to evaluate changes in the quality of life of participants treated with obinutuzumab compared with MMF. The corresponding secondary endpoint is the mean change in "Physical Functioning" domain of PedsQL Quality of Life Inventory from baseline to Week 52.

The fourth secondary objective is to evaluate edema over time. The corresponding secondary end point is the mean change in Cure Glomerulonephropathy (CureGN) Edema Scale from baseline over time to Week 52.

The fifth secondary objective is to evaluate the safety of obinutuzumab compared with MMF. The corresponding secondary endpoints are: (1) the incidence, nature, and severity of adverse events (AEs), with severity determined according to AE intensity (mild, moderate, severe, life-threatening) and National Cancer Institute Common Terminology Criteria for Adverse Events (NCI CTCAE) grading if applicable from baseline to Week 52; and (2) the incidence of laboratory or vital sign abnormalities from baseline to Week 52.

The sixth secondary endpoint is to characterize the obinutuzumab PK profile. The corresponding secondary endpoint is serum concentrations of obinutuzumab at specified timepoints.

The seventh secondary objective is to characterize obinutuzumab-induced PD changes. The corresponding secondary endpoints are: (1) the proportion of participants achieving B cell depletion (*e.g.* using highly sensitive flow cytometry (HSFC)) at specified timepoints; and (2) total peripheral B cell and B cell subsets (*e.g.*, memory B cells) counts and change from baseline at specified timepoints.

An exploratory objective is to evaluate the efficacy of obinutuzumab compared with MMF. The corresponding exploratory endpoints are: (1) change of the UPCR from baseline to Week 52; (2) change of estimated glomerular filtration rate (eGFR) from baseline to Week 52; (3) proportion of participants achieving sustained peripheral B cell depletion at Weeks 24, 52, and 76; (4) proportion of participants who achieve RFS at Week 24; (5) proportion of participants in sustained complete remission at Week 76; (6) change in Physician's Global Assessment of Disease Activity (PGA) from baseline to Weeks 24 and 52; (7) change in Subject Global Assessment of Disease Activity (SGA) from baseline to Weeks 24 and 52; and (8) proportion of participants with no relapses prior to the completion of the steroid taper.

The second exploratory objective is to explore exposure efficacy and exposure safety relationships. The corresponding exploratory endpoints are: (1) change in obinutuzumab exposure and selected efficacy endpoints (including sustainable complete response (SCR) and RFS) from baseline to Week 52 and over time; and (2) change in obinutuzumab exposure and incidence, nature, and severity of selected adverse events from baseline to Week 52 and over time.

The third exploratory objective is to evaluate potential relationships between drug exposure and B cell depletion. The corresponding exploratory endpoints are: (1) change in obinutuzumab exposure and circulating CD19+ B cell counts relative to baseline over time; and (2) total peripheral B cell and B cell subset (*e.g.*, memory B cells) counts prior to/at relapse.

The fourth exploratory objective is to evaluate the immune response to obinutuzumab. The corresponding exploratory endpoint is the proportion of participants with anti-drug antibodies (ADAs) at baseline and incidence of ADAs post treatment during the study.

The fifth exploratory objective is to evaluate potential effects of ADAs. The corresponding exploratory endpoint is the relationship between ADA status and efficacy, safety, PD, or PK endpoints.

The sixth exploratory objective is to identify and/or evaluate biomarkers that provide evidence of obinutuzumab activity (*i.e.*, pharmacodynamic biomarkers) or increase the knowledge and understanding of disease biology and drug safety. The corresponding exploratory endpoint is the relationship between biomarkers in blood and efficacy, safety, PK, immunogenicity, or other biomarker endpoints.

### Inclusion and exclusion criteria

The inclusion criteria for the study include: (1) participant is between ≥ and ≤ 25 years old at the time of randomization; (2) a diagnosis of FRNS or SDNS before the age of 18 years according to international guidelines [*e.g.*, KDIGO 2021, IPNA 2023 (*see* Trautmann et al. (2023) Pediatr Nephrol 38:877-919]; (3) in complete remission defined by the absence of edema, UPCR ≤ 0.2 g/g at screening, and have three consecutive daily urine dipstick readings of trace or negative for protein within the week prior to randomization; (4) has had at least one relapse in the 6 months prior to screening, after discontinuation of or while receiving oral corticosteroids and/or immunosuppressive therapy (*e.g.*, oral cyclophosphamide, levamisole, mizoribine, MMF, or CNIs) to prevent relapses; (5) patients having received cyclophosphamide in the 6 months prior to randomization must have experienced at least 1 relapse subsequent to cyclophosphamide discontinuation; and (6) estimated glomerular filtration rate (eGFR) within normal range for age (by modified Schwartz formula, if less than 18 years of age, or by using the Chronic Kidney Disease Epidemiology Collaboration (CKD-EPI) equation if 18 years of age or older). Frequently relapsing nephrotic syndrome (FRNS) is defined as: ≥ 2 relapses per 6 months within 6 months of disease onset or ≥ 4 relapses per 12 months in any subsequent 12 month. Steroid-dependent nephrotic syndrome (SDNS) is defined as: two consecutive relapses during therapy with prednisone or prednisolone (either at full dose or during tapering) or within 15 days of prednisone or prednisolone discontinuation.

The exclusion criteria include: (1) secondary nephrotic syndrome (*i.e.*, reflux nephropathy, IgA nephropathy, lupus nephritis, *etc.*); (2) history of steroid resistant nephrotic syndrome; (3) history of genetic defects known to directly cause nephrotic syndrome (*i.e.*, NPHS2 [podocin], NPHS1 [nephrin], PLCE1, WT1, or other known genetic cause); (4) treatment with other immunosuppressive medications to prevent relapse, other than MMF or oral corticosteroids within 2 months prior to randomization; (5) history of organ or bone marrow transplant; (6) participation in another therapeutic trial within 30 days of enrollment or 5 half-lives of the investigational drug (whichever is longer); (7) intolerance or contraindication to study therapies, including any of the following: (a) history of severe allergic or anaphylactic reactions to monoclonal antibodies or known hypersensitivity to any component of the obinutuzumab infusion, (b) lack of peripheral venous access, (c) intolerance or contraindication to oral or IV corticosteroids, or (d) intolerance or contraindication to MMF; (8) patients demonstrating prior treatment failure to MMF as defined by two or more relapses in any 6-month period of time while receiving MMF for at least a 6-month duration; (9) participants who, in the judgment of the investigator, are likely to require systemic corticosteroids for reasons other than idiopathic nephrotic syndrome during the study; (10) receipt of an excluded therapy (see below); (11) active infection of any kind (excluding fungal infection of nail beds) or any major episode of infection requiring hospitalization or treatment with IV anti-infective medications within 4 weeks prior to screening, or completion of oral anti-infectives within 2 weeks prior to randomization; (12) evidence of active tuberculosis (TB) infection; (13) History of or currently active primary or secondary immunodeficiency, including known history of HIV infection and other severe Immunodeficiency blood disorders; (14) history of serious recurrent or chronic infection; (15) history of progressive multifocal leukoencephalopathy; (15) history of or current cancer, including solid tumors, hematological malignancies, and carcinoma in situ (except basal cell carcinoma and squamous cell carcinoma of the skin that have been excised and cured) within the past 5 years; (16) major surgery requiring hospitalization during the 4 weeks prior to screening or during screening; (17) high risk for clinically significant bleeding or any condition requiring plasmapheresis, intravenous immunoglobulin, or acute blood product transfusions; (18) evidence of any significant or uncontrolled concomitant disease that, in the investigator's judgment, would preclude participant's participation, including but not limited to nervous system, respiratory, cardiac, hepatic, endocrine, malignant, or gastrointestinal disorders; (19) currently active alcohol or drug abuse or history of alcohol or drug abuse; and (20) any of the following laboratory parameters at screening:
- AST or ALT > 2.5 × upper limit of normal (ULN) (for age and sex) that cannot be attributed to underlying nephrotic syndrome
- Amylase or lipase > 2 × ULN
- Absolute neutrophil count < 1.5 × 10³/µL
- Hemoglobin < 8 g/dL
- Platelet count < 110,000/µL for participants below age 12 and 50,000/µL for patients age 12 or above
- Positive hepatitis B surface antigen
- Positive hepatitis B core antibody
- Positive hepatitis C antibody
- Positive serum human chorionic gonadotropin measured at screening
Excluded therapies include:
- Cyclophosphamide, levamisole, mizoribine, tacrolimus, cyclosporine, or voclosporin during the 2 months prior to screening or during screening.
- Any biologic B cell-depleting therapy (*e*.*g*., anti CD19, anti CD20, anti CD22) such as, but not limited to, rituximab, ocrelizumab, or ofatumumab within 9 months prior to the Day 1 baseline visit
- Any biologic therapy (other than anti-CD19, anti-CD20, anti-CD22) such as, but not limited to belimumab, daratumumab, ustekinumab, anifrolumab, secukinumab, or atacicept during the 2 months prior to screening or during screening
- Oral inhibitors of Janus associated kinase (JAK), Bruton's tyrosine kinase (BTK), or tyrosine kinase 2 (TYK2), including baricitinib, tofacitinib, upadacitinib, filgotinib, ibrutinib, or fenebrutinib or any investigational agent during the 2 months prior to screening or during screening
- Any live vaccine during the 28 days prior to screening or during screening

### Study treatment

The study consists of four periods: a screening period of up to 28 days in length, a 52-week initial open-label treatment period, a post Week 52 extension period, and a minimum 12-month safety follow up (SFU) period that begins at the time of study treatment completion or discontinuation. A study schema is provided in **FIG. 1****.**

Approximately 80 participants aged ≥ 2-25 years old are randomized in a 1:1 ratio to one of two open-label treatment groups: Arm A (obinutuzumab) or Arm B (MMF). Randomization is stratified by the participant's disease type (FRNS vs. SDNS) and the use of immunosuppressive treatment, other than corticosteroids for INS prior to study entry (MMF/other immunosuppressive agent vs. no MMF/other immunosuppressive agent).

The investigational product for the study is obinutuzumab.

After a screening period of 28 days (+/-7 days), randomized participants enter the 52-week treatment period. During the treatment period, participants receive either an infusion of obinutuzumab 1000 mg IV on Days 1, 15, 168 (Week 24), and Day 182 (Week 26), during the initial 52-week treatment period or commence or continue daily oral MMF (either tablet, capsule, or liquid formulation) on Day 1 according to **FIG. 1****.** Participants with a body weight of 45 kg or more receive the 1000 mg obinutuzumab dose. Participants with a body weight below 45 kg receive a weight-adjusted dose of 20 mg/kg for obinutuzumab infusions. Methylprednisolone is administered 80 mg IV (or 1.5mg/kg if ≤45 kg) as premedication prior to infusions. Acetaminophen/paracetamol is administered 15mg/kg (maximum dose 1000mg) PO as predmedication prior to infusions. Diphenhydramine hydrochloride is administered 0.5-1 mg/kg (maximum dose 50mg) PO or IV as premedication prior to infusions.

Participants randomized to MMF take the target dose of 1200 mg/m²/day (maximum 2.5 g/day) in divided doses. Participants who receive daily oral prednisone (or prednisolone equivalent) at randomization taper off to reach the goal of 0 mg/day by Week 8 following randomization (or sooner, *e.g.,* by study Weeks 4-6 if appropriate) and continue without prednisone use through the remainder of the study. Participants who experience disease relapse receive prednisone or prednisolone up to 2 mg/kg/day (60 mg/m²/day, to a max dose of 60 mg/day) until urine protein dipstick is negative/trace for 3 or more consecutive days (or UPCR ≤ 0.2 g/g), followed by a tapering off oral corticosteroids within 4 weeks, while continuing the study treatment regimen. If participants in the MMF arm meet the criteria for rescue therapy, the patient is considered to have met the definition of an intercurrent event and receives obinutuzumab (2 × 1000 mg 14 days apart, or 20 mg/kg if < 45 kg) or alternative therapies for INS. If participants in the obinutuzumab arm meet the criteria for rescue therapy, the patient is considered to have met the definition of an intercurrent event and receives daily MMF (600 mg/m² BID [target 1200 mg/m²] in divided doses, maximum 2 g/day) or alternative therapies to treat the relapse per the investigator's discretion. MMF is discontinued when obinutuzumab is used.

Evaluation of the primary endpoint of the Proportion of Participants with Sustained Complete Remission at 1 year (UPCR ≤ 0.2 g/g without occurrence of a relapse or other intercurrent event) occurs when the last participant randomized completes Week 52.

After the Week 52 assessment, participants can continue with obinutuzumab in the treatment extension period until the common close-out date (CCOD) or directly enter the safety follow-up (SFU). Participants, whether in the MMF or obinutuzumab arms, who exhibit relapse after Week 52 are treated as per the best judgment of the investigator, which can include administration of initial or additional doses of obinutuzumab. Patients who do not meet the criteria for relapse on or after Week 52 are not eligible for obinutuzumab treatment in the extension period, but are followed every 3 months at study visits until CCOD. Participants with peripheral B cell depletion are followed in SFU every 12 weeks for 6 months, then every 6 months thereafter until return of peripheral CD19 B cells to the pre-treatment value or to within the central laboratory normal range for this patient population, whichever is lower, until the end of study.

The first SFU visit is scheduled approximately 12 weeks after the last study visit in the treatment period or CCOD, whichever occurs first. No obinutuzumab infusions or MMF are provided during SFU. Standard of care therapies are provided at the investigator's discretion. Patients who do not receive obinutuzumab are seen for only a single SFU visit, with assessments specified in SFU Visit 1. Patients who receive obinutuzumab are followed in SFU until such patients fulfill both of the following criteria: (1) peripheral B cells have returned to pre-obinutuzumab baseline levels or to within the normal range for the population, whichever is lower; and (2) the last infusion of obinutuzumab was at least 12 months ago.

At each SFU visit, the absolute CD19+ B-cell count is measured. For participants with persistent B-cell depletion, defined as an absolute CD19+ B-cell count below the lowest pretreatment value and less than the lower limit of normal (LLN) for this population, and who have not received an additional therapy associated with reductions in peripheral B cells, SFU will continue every 6 months until any of the following occurs: (1) return of peripheral CD19+ B cells to the lowest pretreatment value or the age specific LLN for the patient population, whichever is lower; (2) receipt of an additional therapy associated with reductions in peripheral B cells (e.g., belimumab, rituximab, or cyclophosphamide, or use of obinutuzumab outside the study protocol); or (3) the study ends.

Participants complete the study when: (1) the SFU requirements are completed; (2) the last study visit of treatment on or after CCOD is completed and the investigator intends to treat the participant for nephrotic syndrome outside of the study protocol without completing the required SFU; (3) or the study ends. Continued access to Roche investigational medicinal product (IMP; obinutuzumab) is offered to eligible participants by the study Sponsor upon completion of the study.

### Duration of study period

The minimum duration of study participation for each individual is expected to be approximately 1.5-2 years (SFU will be a minimum of 12 months after the last obinutuzumab infusion, for all patients who have received obinutuzumab). Participants may continue to participate in the study and be followed up or receive repeat treatment according to the Schedule of Activities until the last participant recruited has participated in the study for at least 18 months.

The maximum duration of study participation is estimated to be approximately 3 years, or longer if peripheral B cells remain below LLN at the clinical cut off. In this case, participants will be required to return for SFU visits every 12 weeks for 6 months, then every 6 months thereafter until B cells return to pre obinutuzumab dose baseline or the central laboratory LLN of normal for the participant's population, or until the study ends.

## Claims

1. A type II anti-CD20 antibody for use in a method of treating, or reducing risk and/or frequency of relapse of, childhood-onset idiopathic nephrotic syndrome (INS) in an individual in need thereof, wherein the type II anti-CD20 antibody is obinutuzumab; wherein the method comprises administering to the individual a first antibody exposure to the type II anti-CD20 antibody and a second antibody exposure to the type II anti-CD20 antibody;
wherein the second antibody exposure is not being provided until from about 18 weeks to about 26 weeks after the first antibody exposure;
wherein the first antibody exposure comprises one or two doses of the type II anti-CD20 antibody, the first antibody exposure comprising:
(a) a total exposure of between about 1800 mg and about 2200 mg of the type II anti-CD20 antibody, or
(b) a total exposure of between about 36 mg/kg and about 44 mg/kg of the type II anti-CD20 antibody if the individual weighs less than 45 kg;
wherein the second antibody exposure comprises one or two doses of the type II anti-CD20 antibody, the second antibody exposure comprising:
(c) a total exposure of between about 1800 mg and about 2200 mg of the type II anti-CD20 antibody, or
(d) a total exposure of between about 36 mg/kg and about 44 mg/kg of the type II anti-CD20 antibody if the individual weighs less than 45 kg;
and wherein the individual is a human that is greater than or equal to 2 years of age and less than or equal to 25 years of age.

2. The type II anti-CD20 antibody for use according to claim 1, wherein the first antibody exposure comprises a first dose of between about 900 mg and about 1100 mg and a second dose of between about 900 mg and about 1100 mg; or wherein the first antibody exposure comprises a first dose of between about 18 mg/kg and about 22 mg/kg and a second dose of between about 18 mg/kg and about 22 mg/kg, and wherein the individual weighs less than 45 kg.

3. The type II anti-CD20 antibody for use according to claim 1 or claim 2, wherein the second antibody exposure comprises a first dose of between about 900 mg and about 1100 mg and a second dose of between about 900 mg and about 1100 mg; or wherein the second antibody exposure comprises a first dose of between about 18 mg/kg and about 22 mg/kg and a second dose of between about 18 mg/kg and about 22 mg/kg, and wherein the individual weighs less than 45 kg.

4. The type II anti-CD20 antibody for use according to any one of claims 1 to 3, wherein the first antibody exposure and/or the second antibody exposure comprises a first dose and a second dose, and wherein the second dose is not provided until from about 1.5 weeks to about 2.5 weeks after the first dose.

5. The type II anti-CD20 antibody for use according to claim 4, wherein the second dose is not provided until about 2 weeks after the first dose.

6. The type II anti-CD20 antibody for use according to any one of claims 1 to 5, wherein the doses of the first and/or second antibody exposure are about 1000 mg; or wherein the doses of the first and/or second antibody exposure are about 20 mg/kg, and wherein the individual weighs less than 45 kg.

7. The type II anti-CD20 antibody for use according to any one of claims 1 to 6, wherein the first antibody exposure comprises two doses of 1000 mg or 20 mg/kg on weeks 0 and 2 of treatment; and wherein the second antibody exposure comprises two doses of 1000 mg or 20 mg/kg on weeks 24 and 26 of treatment.

8. The type II anti-CD20 antibody for use according to any one of claims 1 to 7, wherein the childhood-onset INS is frequently relapsing nephrotic syndrome (FRNS).

9. The type II anti-CD20 antibody for use according to any one of claims 1 to 7, wherein the childhood-onset INS is steroid-dependent nephrotic syndrome (SDNS).

10. The type II anti-CD20 antibody for use according to any one of claims 1 to 9, wherein the individual is in complete remission prior to the administration.

11. The type II anti-CD20 antibody for use according to any one of claims 1 to 10, wherein the method further comprises administering to the individual an effective amount of a glucocorticoid or corticosteroid.

12. The type II anti-CD20 antibody for use according to claim 11, wherein the glucocorticoid or corticosteroid comprises methylprednisolone; optionally wherein the methylprednisolone is administered intravenously at a dose of 80 mg, or intravenously at a dose of 1.5 mg/kg if the individual weighs less than 45 kg.

13. The type II anti-CD20 antibody for use according to any one of claims 1 to 12, wherein the method further comprises administering to the individual an effective amount of an antihistamine, optionally wherein the antihistamine comprises diphenhydramine; and/or an effective amount of acetaminophen.

14. The type II anti-CD20 antibody for use according to any one of claims 1 to 13, wherein the method results in a sustained complete remission in the individual at 1 year.

15. A kit for treating childhood-onset INS in an individual, comprising: (a) a container comprising a type II anti-CD20 antibody, wherein the type II anti-CD20 antibody is obinutuzumab; (b) a package insert with instructions for treating childhood-onset INS in an individual, wherein the instructions indicate that the individual is a human that is greater than or equal to 2 years of age and less than or equal to 25 years of age; and wherein the instructions further indicate that a first antibody exposure to the type II anti-CD20 antibody and a second antibody exposure to the type II anti-CD20 antibody are administered to the individual, the second antibody exposure not being provided until from about 18 weeks to about 26 weeks after the first antibody exposure; wherein the first antibody exposure comprises a total exposure of between about 1800 mg and about 2200 mg, or between about 36 mg/kg and about 44 mg/kg if the individual weighs less than 45 kg; and wherein the second antibody exposure comprises a total exposure of between about 1800 mg and about 2200 mg, or between about 36 mg/kg and about 44 mg/kg if the individual weighs less than 45 kg.
